# EUROPEAN PATENT SPECIFICATION

(11) **EP 3 519 814 B1**
(45) Date of publication and mention of the grant of the patent: **02.06.2021**
(21) Application number: 17794809.8
(22) Date of filing: 02.10.2017
(51) Int. Cl.: G01N 33/50

(54) **METABOLIC RESTRICTION IN CELL-BASED ASSAYS**
METABOLISCHE EINSCHRÄNKUNG BEI TESTS AUF ZELLBASIS
RESTRICTION MÉTABOLIQUE DANS DES DOSAGES BASÉ SUR DES CELLULES

(30) Priority: 30.09.2016 US 201662402534 P
(43) Date of publication of application: 07.08.2019
(73) Proprietor: Novira Therapeutics, Inc., Doylestown PA 18902 (US)
(72) Inventor: LAM, Man Iu, Spring House, PA 19477 (US); ESPIRITU, Christine Lisa, Spring House, PA 19477 (US); KUDUK, Scott, Spring House, PA 19477 (US); HARTMAN, George D., Lansdale, PA 19446 (US)
(74) Representative: Daelemans, Frank F.R.
(86) International application number: PCT/US2017/054782
(87) International publication number: WO 2018/064675

(56) References cited:
- WO-A1-99/12032
- WO-A1-99/12032
- WO-A1-2014/061244
- WO-A1-2014/061244
- CONGRONG NIU ET AL: "Metabolic Activation of the Anti-Hepatitis C Virus Nucleotide Prodrug PSI-352938", ANTIMICROBIAL AGENTS AND CHEMOTHERAPY, vol. 56, no. 7, 23 April 2012 (2012-04-23) , pages 3767-3775, XP055446083, ISSN: 0066-4804, DOI: 10.1128/AAC.00530-12
- M. R. CAMPAGNA ET AL: "Sulfamoylbenzamide Derivatives Inhibit the Assembly of Hepatitis B Virus Nucleocapsids", JOURNAL OF VIROLOGY, vol. 87, no. 12, 10 April 2013 (2013-04-10), pages 6931-6942, XP055153462, ISSN: 0022-538X, DOI: 10.1128/JVI.00582-13
- M. GOMEZ-LECHON ET AL: "Human Hepatocytes in Primary Culture: The Choice to Investigate Drug Metabolism in Man", CURRENT DRUG METABOLISM, vol. 5, no. 5, 1 October 2004 (2004-10-01) , pages 443-462, XP055445748, US ISSN: 1389-2002, DOI: 10.2174/1389200043335414
- CONGRONG NIU ET AL: "Metabolic Activation of the Anti-Hepatitis C Virus Nucleotide Prodrug PSI-352938", ANTIMICROBIAL AGENTS AND CHEMOTHERAPY, vol. 56, no. 7, 23 April 2012 (2012-04-23) , pages 3767-3775, XP055446083, US ISSN: 0066-4804, DOI: 10.1128/AAC.00530-12
- M. R. CAMPAGNA ET AL: "Sulfamoylbenzamide Derivatives Inhibit the Assembly of Hepatitis B Virus Nucleocapsids", JOURNAL OF VIROLOGY, vol. 87, no. 12, 10 April 2013 (2013-04-10), pages 6931-6942, XP055153462, ISSN: 0022-538X, DOI: 10.1128/JVI.00582-13
- M. GOMEZ-LECHON ET AL: "Human Hepatocytes in Primary Culture: The Choice to Investigate Drug Metabolism in Man", CURRENT DRUG METABOLISM, vol. 5, no. 5, 1 October 2004 (2004-10-01) , pages 443-462, XP055445748, US ISSN: 1389-2002, DOI: 10.2174/1389200043335414

## Description

### BACKGROUND

There exist a high number of diseases that can negatively impair the liver and its function. These liver diseases can be genetic or caused by a variety of factors such as viruses, alcohol abuse, and obesity, all of which can cause the liver to become inflamed. This inflammation can progress to scarring or cirrhosis which increases the likelihood of developing liver cancer or liver failure. More than 300,000 people are hospitalized each year due to cirrhosis, and thousands die each year from chronic liver disease.

Hepatocyte assays are useful for the identification of agents that can be used in the treatment of diseases of, or related to, the liver, including malaria, diabetes, viral hepatitis, such as, for example, HBV infection, nonalcoholic steatohepatitis, fatty liver disease, and cirrhosis. These assays are essential to accurately assess the efficacy of a potential treatment agent. Under standard assay conditions, metabolically active cells, such as hepatocytes, can metabolize agents being tested for certain functional activity such as HBV antiviral activity such that any observed antiviral effects can be the result of a pool of metabolites and not the test agent itself. Disrupting the natural ability of a hepatocyte to metabolize agents being tested can also affect the functional activity of the hepatocyte serving as the endpoint for the tested agent. The presence of metabolites of the tested agent and disruption of the natural functional activity of hepatocytes presents significant technical problems in accurately measuring endpoints in assays used to screen agents for treatment of liver disease.

Hepatitis B virus (HBV) infection is a liver disease of particular concern due to its devastating effects on the liver. HBV infection is a significant global health problem, estimated to affect over 3% of the world population (over 240 million people worldwide).

Despite the availability of a prophylactic HBV vaccine, the burden of chronic HBV infection continues to be a significant unmet worldwide medical problem, due to suboptimal treatment options and sustained rates of new infections in most parts of the developing world. Current treatments do not provide a cure and are limited to only two classes of agents (interferon alpha and nucleoside analogues/inhibitors of the viral polymerase); drug resistance, low efficacy, and tolerability issues limit their impact. The low cure rates of HBV infection are attributed at least in part to the fact that complete suppression of virus production is difficult to achieve with a single antiviral agent. Current therapy goals for HBV-infected patients are directed to reducing serum HBV DNA to low or undetectable levels and to ultimately reducing or preventing the development of cirrhosis and hepatocellular carcinoma The persistent epigenetic silencing or the clearance of cccDNA can enable the persistent suppression of HBV replication without requiring ongoing treatmen.

M. R. CAMPAGNA ET AL: "Sulfamoylbenzamide Derivatives Inhibit the Assembly of Hepatitis B Virus Nucleocapsids", JOURNAL OF VIROLOGY, vol. 87, no. 12, 10 April 2013 (2013-04-10), pages 6931-6942 and WO 99/12032 A1 disclose cellular screening assays for identifying anti-HBV compounds. There is, therefore, a need in the art not only for therapeutic agents that can successfully prevent, treat, or ameliorate liver disease such as HBV, but there is also a need in the art for methods to accurately measure endpoints in assays used to screen agents for treatment of liver disease.

### SUMMARY OF INVENTION

Provided herein are methods and assays for identifying and optimizing agents for the treatment of liver disease. The disclosure also includes methods of modulating (e.g., inhibiting) the natural ability of cells having a metabolic capacity, such as hepatocytes, to metabolize test agents without disrupting the ability to measure or determine the achievement by the test agent of a functional effect or endpoint in the hepatocyte. The methods disclosed herein enable the screening of test agents in an assay using cells with a metabolic capacity while the cell viability and the ability of achieving assay endpoints is maintained.

Accordingly, provided herein are methods of reducing the metabolism of a test agent in a primary human hepatocyte (PHH), comprising the steps of, in any order:
(a) treating the PHH with an enzyme modulator; and
(b) applying the test agent to the PHH.

Treating the PHH with an enzyme modulator modulates (e.g., increases or decrease) the metabolic capacity of the PHH. The PHH can be treated with one or more enzyme modulators. In embodiments, the PHH is treated with one or more enzyme modulators before applying the test agent to the PHH. In embodiments, the PHH is treated with one or more enzyme modulators after applying the test agent to the PHH. In embodiments, the PHH is treated with one or more enzyme modulators before and after applying the test agent to the PHH. In an embodiment, the enzyme modulator modulates the metabolic capacity of the PHH.

In another aspect, provided herein are methods of identifying a liver-related disease treatment test agent in a primary human hepatocyte, comprising the steps of, in any order:
(a) treating a PHH with enzyme modulator; and
(b) applying a test agent to the PHH.

In an embodiment, to suppress metabolism, hepatocytes can be incubated with one or more CYP inhibitors. CYP inhibitors can be combined in a diluent to achieve a desired concentration. CYP inhibitors can also be allowed to incubate with the hepatocytes. After CYP inhibitor incubation, media containing CYP inhibitors can be removed, and test agents in diluent can be added to hepatocytes and allowed to incubate for a set amount of time. After incubation, test agents can be removed and hepatocytes can be incubated with freshly prepared CYP inhibitors. After CYP inhibitors incubation, media containing CYP inhibitors can be removed and test agents in diluent can be added to hepatocytes for an additional predetermined amount of time. Cells treated with diluent alone can serve as no test agent control. Analytical assays, enzyme inhibition, and cell viability assays can be performed after test agent treatment.

Also provided herein are methods and assays for identifying and optimizing hepatitis B virus antiviral agents. Antiviral test agents are susceptible to being metabolized by HBV-infected cells under standard assay conditions. By modulating (e.g., inhibiting) the ability of the PHH to metabolize the test agents, the test agents are minimally metabolized or not metabolized when applied to the PHH, and their effect on hepatocyte activity can be detected and measured.

In another aspect, provided herein are methods of identifying a hepatitis B virus (HBV) antiviral agent, wherein the assay comprises an HBV-infected cell, wherein the metabolic capacity of the HBV-infected cell is or will be modulated, and applying a test agent to the HBV-infected cell such that the test agent is not metabolized or is minimally metabolized during the assay. In an embodiment, the metabolic capacity of the HBV-infected cell is modulated after infecting the cell with HBV. In another embodiment, the metabolic capacity of the HBV-infected cell is modulated before applying the test agent. In an embodiment, the HBV-infected cell is treated with one or more enzyme modulators that modulate the metabolic capacity of the HBV-infected cell. In another embodiment, the viability of the modulated cell is maintained. In an embodiment, an assay endpoint is maintained. In yet other embodiments, the modulation of the cell metabolic capacity is accomplished by contacting the cell with one or more cytochrome P450 inhibitors. Non-limiting examples of cytochrome P450 inhibitors that can be used in the assay include SKF-525A and 1-aminobenzotriazole.

In one aspect, disclosed herein are methods of identifying a hepatitis B virus (HBV) antiviral agent in an assay, wherein the assay comprises applying a test agent to an HBV-infected cell, wherein the HBV-infected cell is treated with one or more cytochrome P450 inhibitors that modulate or inhibit the metabolic capacity of the HBV-infected cell such that the test agent is not metabolized or is minimally metabolized during the assay, and wherein cell viability and an assay endpoint is maintained.

In another aspect, disclosed herein are methods of identifying a hepatitis B virus (HBV) antiviral agent in an assay, wherein the assay comprises applying a test agent to an HBV-infected cell, wherein the metabolic capacity of the HBV-infected cell is or will be modulated such that the test agent is not metabolized or is minimally metabolized during the assay, comprising the steps of, in any order:
(a) treating a cell with one or more enzyme modulators that modulate the metabolic capacity of the cell;
(b) infecting the cell with HBV; and
(c) applying the test agent to the HBV-infected cell.

In disclosed embodiments, the viability of the modulated cell is maintained. In other embodiments, assay endpoint is maintained. In yet other embodiments, the metabolic capacity of the HBV-infected cell is modulated after infecting the cell with HBV. In other embodiments, the metabolic capacity of the HBV-infected cell is modulated before applying the test agent. In yet other embodiments, the one or more enzyme modulators comprise cytochrome P450 inhibitors.

An assay endpoint is a point during the assay where a substance or an activity (or cell viability) is measured. In embodiments of the disclosed methods, the assay endpoint can be a substance or an event in the life-cycle of an HBV virus that can be measured, such as antiviral innate immune genes expression and/or protein production, antiviral genes expression and/or protein production, HBV cccDNA, degraded HBV cccDNA, transcription of HBV cccDNA, nuclear import of HBV polypeptides or HBV DNA, nuclear export of HBV polypeptides or HBV DNA, a function of HBV polypeptide, a function of HBV core polypeptide, HBV capsid polypeptide assembly, HBV replication, HBV genome encapsidation, HBV genome replication, HBV RNA encapsidation, HBV RNA secretion, HBV RNA splicing, HBV virion morphogenesis, HBV virion secretion, HBsAg presence, and HBeAg presence. In other embodiments, the assay endpoint is cell viability.

In other embodiments, the test agent is identified as a hepatitis B virus (HBV) antiviral agent when the appropriate HBV assay endpoints are compared to a control cell in the assay lacking the test agent.

In disclosed embodiments, the one or more enzyme modulators is a cytochrome P450 inhibitor. In embodiments, the enzyme modulator is selected from the group consisting of 1-aminobenzotriazole, abiraterone, amiodarone, amprenavir, anastrozole, aprepitant, asenapine, atazanavir, atazanavir, boceprevir, buproprion, celecoxib, chloroquine, chlorpheniramine, chlorpromazine, cimetidine, cinacalcet, ciprofloxacin, citalopram, clarithromycin, clemastine, clomipramine, clopidogrel, cocaine, contraceptives, cotrimoxazole, cyclosporine, danazol, delavirdine, desipramine, diltiazem, diphenhydramine, disulfiram, doxepin, doxorubicin, duloxetine, efavirenz, enoxacin, erythromycin, escitalopram, estradiol, ethinyl estradiol, ezetimibe (p), febuxostat, felbamate, fenofibrate, fluconazole, fluorouracil, fluoxetine, fluphenazine, fluvastatin, fluvoxamine, gemfibrozil, gestodene, halofantrine, haloperidol, hydroxychloroquine, hydroxyzine, imatinib, indinavir, indomethacin, interferon, isoniazid, isoniazid ketoconazole, itraconazole, ketoconazole, lansoprazole, leflunomide, levomepromazine, lovastatin, methadone, methoxsalen, methylprednisolone, metoclopramide, metronidazole, mexiletine, mibefradil, miconazole, midodrine, mifepristone, moclobemide, modafinil, montelukast, nalidixic acid, nefazodone, nelfinavir, nicardipine, nifedipine, norethindrone, norfloxacin, norfluoxetine, omperazole, oxcarbazepine, oxiconazole, paroxetine, perphenazine, phenylbutazone, posaconazole, prednisone, proadifen hydrochloride (SKF-525A), probenecid, propafenone, propoxyphene, propranolol, quinacrine, quinidine, quinine, ranitidine, ranolazine, ritonavir, roxithromycin, saquinavir, sertraline, sulfamethoxazole, sulfaphenazole, sulfinpyrazone, sulfonamides, tacrine, tegaserod, telaprevir, telithromycin, teniposide, terbinafine, thiopeta, thioridazine, ticlodipine, tipranavir, topiramate, trimethoprim, tripelennamine, troleandomycin, verapamil, voriconazole, zafirlukast, and zileutin. In an embodiment of a disclosed aspect, the cytochrome P450 inhibitor is selected from this same group. In embodiments the one or more cytochrome P450 inhibitors are SKF-525A and 1-aminobenzotriazole.

In disclosed embodiments, the cell can be any one of a human, a liver cell, a differentiated liver cell, and a cell from a cell line or a primary culture cell. In embodiments wherein the cell is from a cell line, it can be a HepaRG cell; in those embodiments wherein the cell is a primary culture cell, the cell can be a primary hepatocyte, such as a primary human hepatocyte or a cryopreserved primary human hepatocyte. In embodiments wherein the cell is from an inducible pluripotent stem cell differentiated into hepatocyte-like cell, the cell can be an induced pluripotent stem cell-derived hepatocyte. In embodiments, the cell can comprise a sodium taurocholate co-transporting polypeptide (NTCP) receptor for the HBV; in some embodiments, the NTCP receptor is exogenously expressed.

Also disclosed herein are methods of identifying an antiparasitic agent in an assay, wherein the assay comprises applying a test agent to a *Plasmodium-*infected cell, wherein the metabolic capacity of the *Plasmodium-*infected cell is or will be modulated such that the test agent is not metabolized or is minimally metabolized during the assay, comprising the steps of, in any order:
(a) treating a cell with one or more enzyme modulators that modulate the metabolic capacity of the cell;
(b) infecting the cell with *Plasmodium;* and
(c) applying the test agent to the *Plasmodium-infected* cell.

In an embodiment, the viability of the modulated cell is maintained. In another embodiment, an assay endpoint is maintained. In yet another embodiment, the metabolic capacity of the *Plasmodium-*infected cell is modulated after infecting the cell with *Plasmodium.* In an embodiment, the metabolic capacity of the *Plasmodium-*infected cell is modulated before applying the test agent. In yet another embodiment, the assay endpoint is the maturation of sporozoites. In another embodiment, the test agent is identified as an anti-parasitic agent when the appropriate *Plasmodium* assay endpoints are compared to a control cell in the assay lacking the test agent.

In still another aspect, disclosed herein are methods of identifying an antidiabetic agent in an assay, wherein the metabolic capacity of the cell is or will be modulated such that the test agent is not metabolized or is minimally metabolized during the assay, comprising the steps of, in any order:
(a) treating a cell with one or more enzyme modulators that modulate the metabolic capacity of the cell; and
(b) applying the test agent to the cell.

In an embodiment, the viability of the modulated cell is maintained. In yet another embodiment, an assay endpoint is maintained. In another embodiment, the assay endpoint is hepatic glucose production. In yet another embodiment, the assay endpoint is AMP-activated protein kinase activity. In another embodiment, the test agent is identified as an antidiabetic agent when the appropriate assay endpoints are compared to a control cell in the assay lacking the test agent.

Disclosed herein is an assay comprising at least one metabolizing cell, at least one enzyme modulator, and at least one test agent metabolized by the metabolizing cell. In an embodiment, the metabolizing cell is a hepatocyte. In yet another embodiment, the hepatocyte is infected with HBV. In another embodiment, the test agent is an antiviral agent. In yet another embodiment, the hepatocyte is infected with *Plasmodium.* In another embodiment, the test agent is an antiparasitic agent. In yet another embodiment, the test agent is an antidiabetic agent. In an embodiment, the enzyme modulator inhibits a cytochrome P450 enzyme. In yet another embodiment, the enzyme modulator comprises a first enzyme modulator and a second enzyme modulator. In another embodiment, the first enzyme modulator is proadifen hydrochloride and the second enzyme modulator is 1-aminobenzotriazole. In a further embodiment, the concentration of proadifen hydrochloride is 2 µM to 100 µM. In yet another embodiment, the concentration of 1-aminobenzotriazole is 5mM to 20mM.

In another aspect, disclosed herein is a method of identifying a hepatitis B virus (HBV) antiviral agent comprising:
(a) treating a metabolizing cell with one or more enzyme modulators;
(b) infecting the metabolizing cell with HBV;
(c) applying a test agent to the HBV-infected cell; and
(d) determining if the test agent achieves an HBV endpoint, wherein achievement of the HBV endpoint identifies the test agent as an HBV antiviral agent.

### BRIEF DESCRIPTION OF THE FIGURES

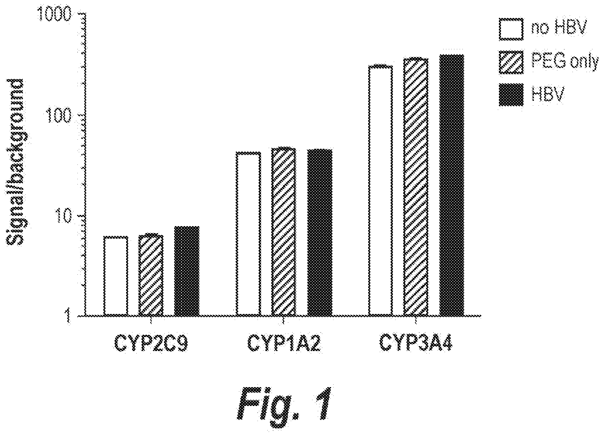

Figure 1 shows a graph summarizing the effect of HBV infection on CYP2C9, CYP1A2, and CYP3A4 enzyme activities in HBV-infected HepaRG cells.

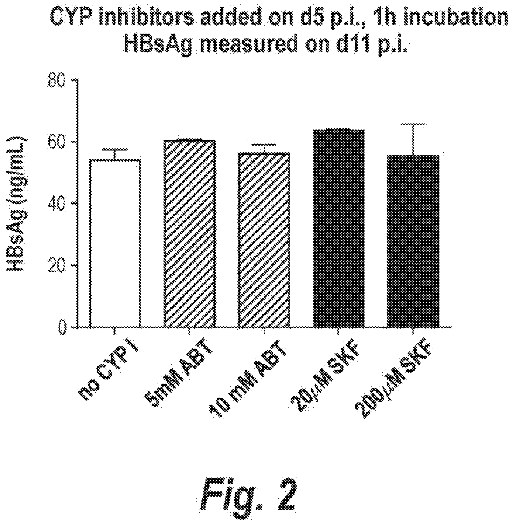

Figure 2 shows a graph summarizing the results of treating HBV-infected HepaRG cells with cytochrome P (CYP) inhibitors 1-aminobenzotrazole (ABT) and SKF-525A ("SKF") and measuring HBsAg secretion. The CYP inhibitors were added for 1 hour on day 5 post infection (p.i.), and HBsAg secretion measured on day 11 post infection (p.i.)

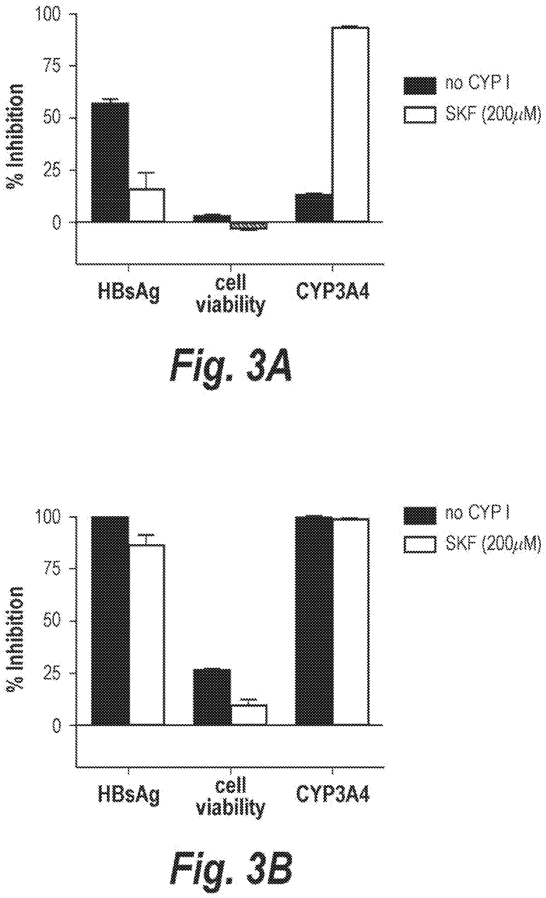

Figures 3A - 3B show graphs summarizing the results of the effects of 200 µM SKF-525A on the activity of an HBV antiviral agent, HAA-1 at two different concentrations (50 µM, Fig. 3A and 200 µM, Fig. 3B), in HBV-infected HepaRG cells. HBsAg secretion, cell viability, and CYP3A4 activity were measured.

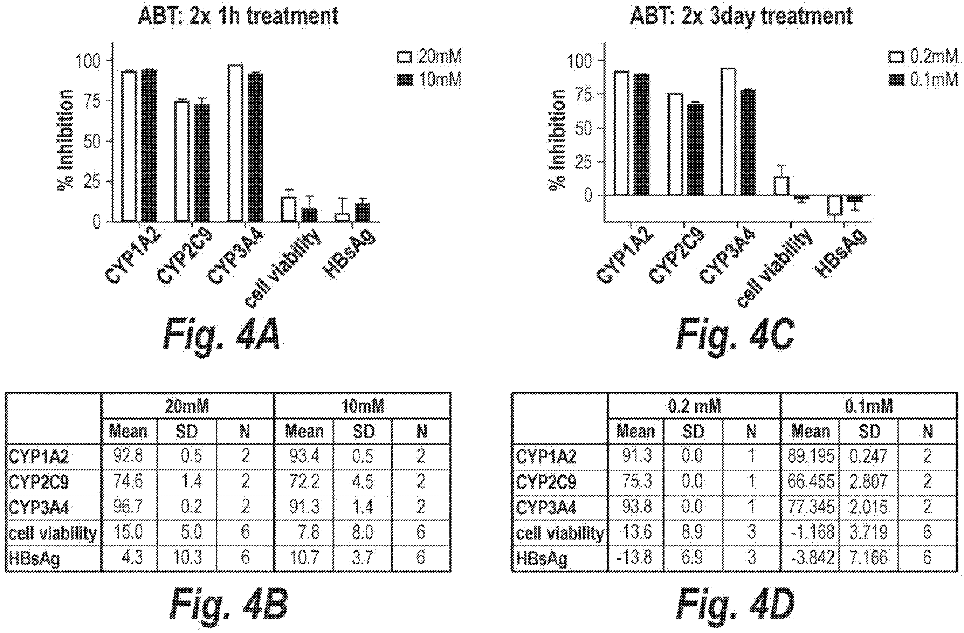

Figures 4A - 4D show graphical depictions (Figs. 4A, 4C) of the data found in the tables (Figs. 4B, 4D), which present the results of the effect of ABT treatment at the indicated concentrations, either applied for one hour and then removed (repeated once for a total of two times) (days 5 and 8 p.i.; Figs. 4A, 4B) or continuously applied for three days (days 5 and 8 p.i.; Figs. 4C, 4D), on HBV-infected HepaRG cells. For continuous application, ABT was added to infected cells on day 5 post-infection. Cells were incubated with compound for 3 days, after which medium was removed and replaced with fresh medium containing ABT and cells were treated for an additional 3 days. The indicated CYP enzyme activities, cell viability and HBsAg secretion were measured on day 11 p.i.

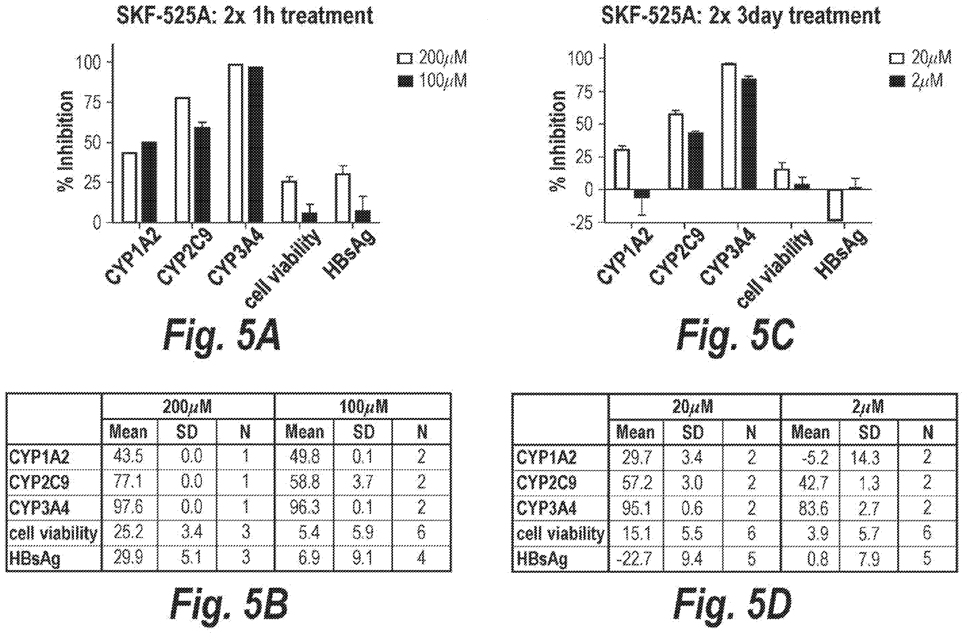

Figures 5A - 5D show graphical depictions (Figs. 5A, 5C) of the data found in the tables (Figs. 5B, 5D) which present the results of the effect of SKF-525A treatment at the indicated concentrations, either applied for one hour and then removed (repeated once for a total of two times) (days 5 and 8 p.i.) (Figs. 5A, 5B) or continuously applied for three days (days 5 and 8 p.i.; Figs. 5C, 5D) on HBV-infected HepaRG cells. For continuous application, SKF was added to infected cells on day 5 post infection. Cells were incubated with compound for 3 days, after which medium was removed and replaced with fresh medium containing SKF and cells were treated for an additional 3 days. The indicated CYP enzyme activities, cell viability and HBsAg secretion were measured on day 11 p.i.

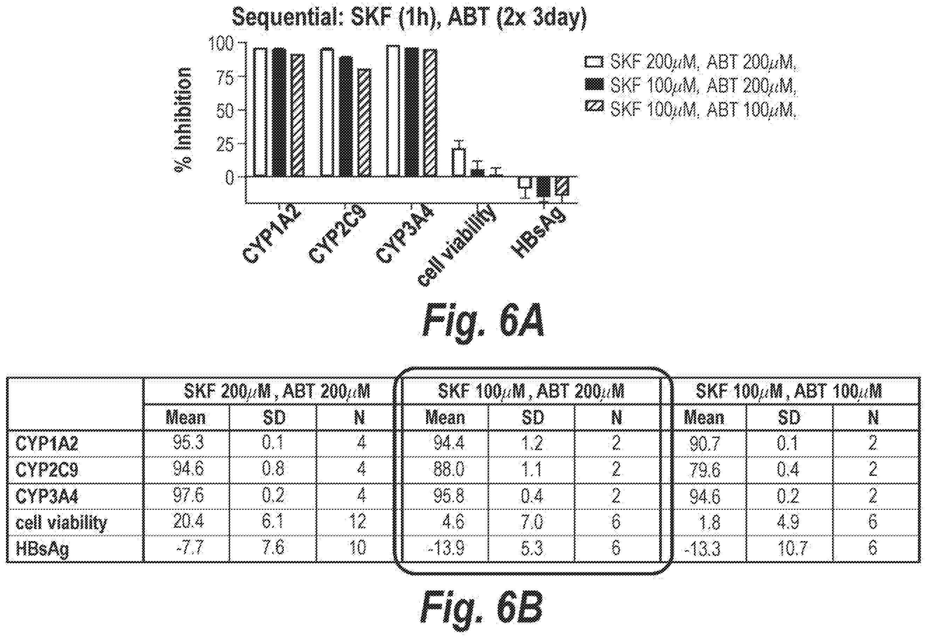

Figures 6A - 6B show a graph (Fig. 6A) of the data found in the table of Fig. 6B presenting the results of sequential treatment of HBV-infected HepaRG cells with SKF-525A and ABT in the sequence and concentrations indicated. The assay endpoints were measured on day 11 p.i.

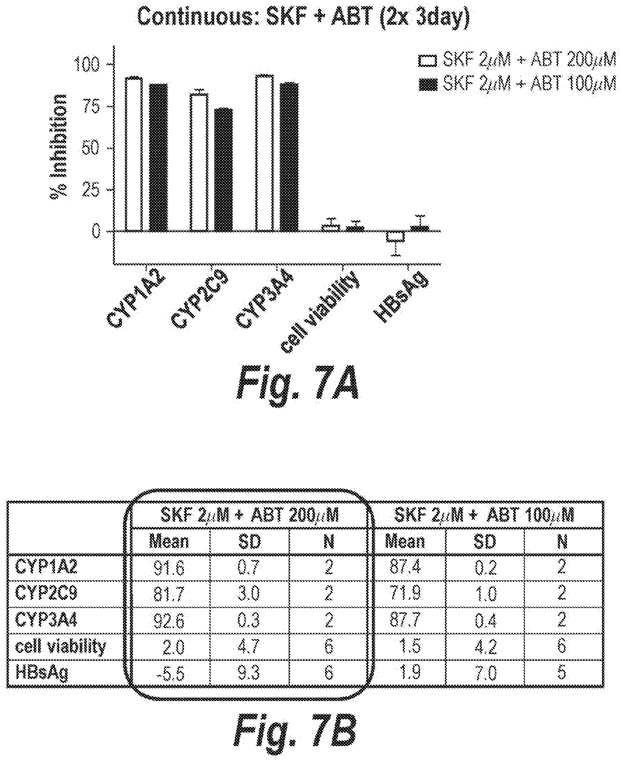

Figures 7A - 7B show a graph (Fig. 7A) of the data found in the table of Fig. 7B presenting the results of continuous, combined treatment of HBV-infected HepaRG cells with SKF-525A and ABT at the concentrations indicated. Treatments with the CYP inhibitors began on day 5 p.i., and were reapplied on day 8 p.i. The assay endpoints were measured on day 11 p.i.

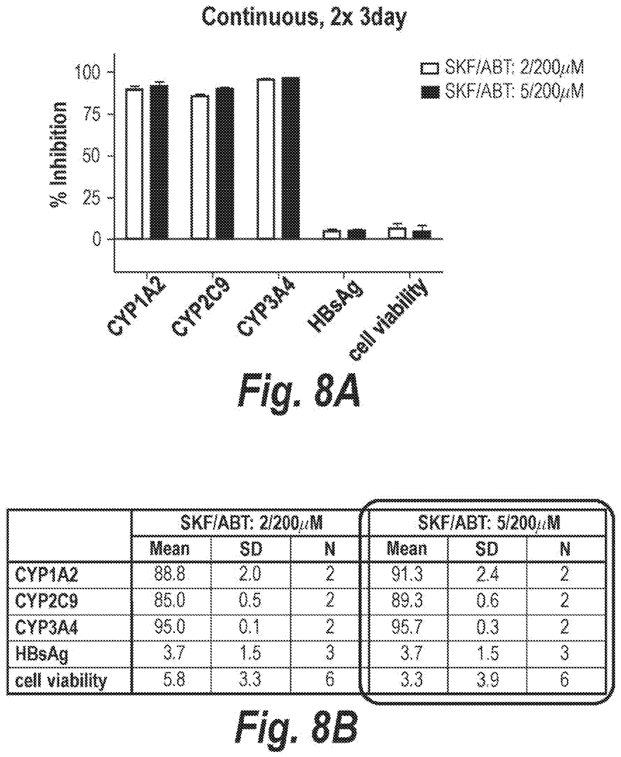

Figures 8A - 8B show a graph (Fig. 8A) of the data found in the table of Fig. 8B of the results of continuous treatment with SKF-525A and ABT (combined) at the indicated concentrations of HBV-infected HepaRG cells. The CYP inhibitors were applied on days 5 and 8 p.i., and the assay endpoints measured on day 11 p.i.

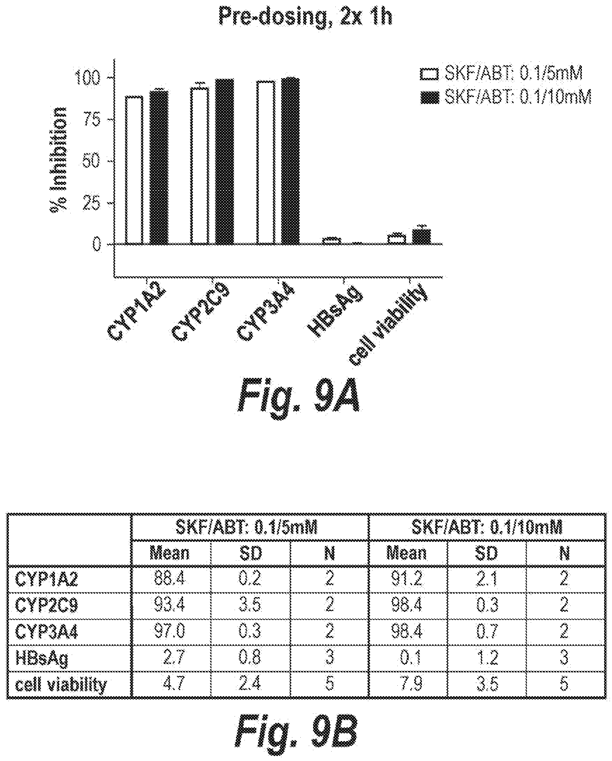

Figures 9A - 9B show a graph (Fig. 9A) of the data found in the table of Fig. 9B of the results of a pre-dosing regimen of applying SKF-525A and ABT in combination at the concentrations indicated. The CYP inhibitors were applied to HBV-infected HepaRG cells for 1 hour at days 5 and 8 p.i., and the assay endpoints measured on day 11 p.i.

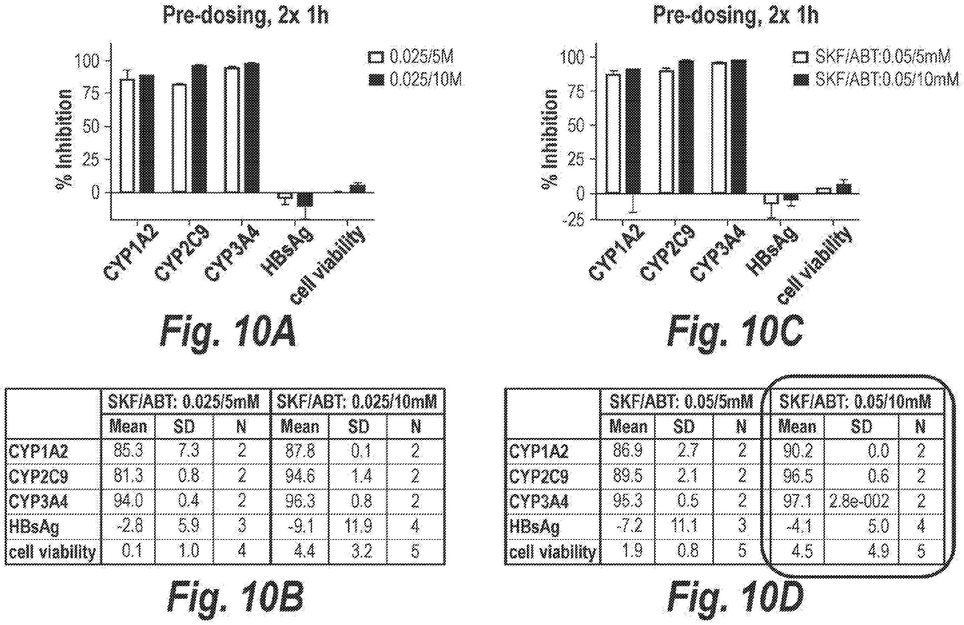

Figures 10A -10D show graphs (Figs. 10A, 10C) of the data found in the tables of Figs 10B, 10D, respectively of the results of a pre-dosing regimen using combinations of SKF-525A and ABT at the indicated concentrations. The CYP inhibitors were applied to HBV-infected HepaRG cells for 1 hour at days 5 and 8 p.i., and the assay endpoints measured on day 11 p.i.

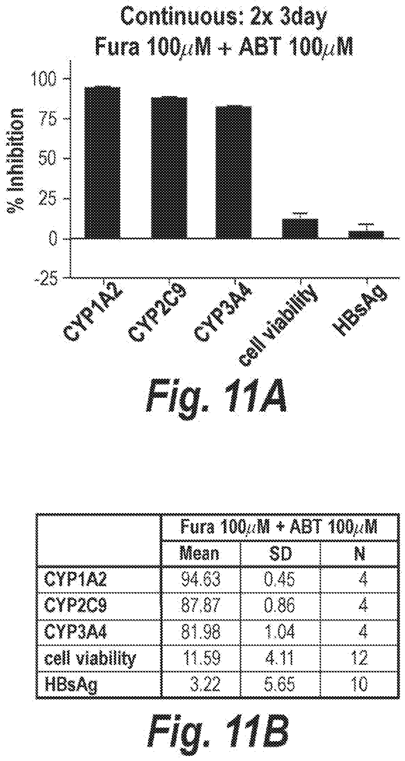

Figures 11A - 11B show a graph (Fig. 11A) of the data found in the table of Fig. 11B of continuous CYP inhibitor treatment with the CYP1A2-selective inhibitor furafylline in combination with ABT (both at 100 µM) of HBV-infected HepaRG cells. The CYP inhibitors were applied on days 5 and 8 p.i. and the assay endpoints measured at day 11 p.i.

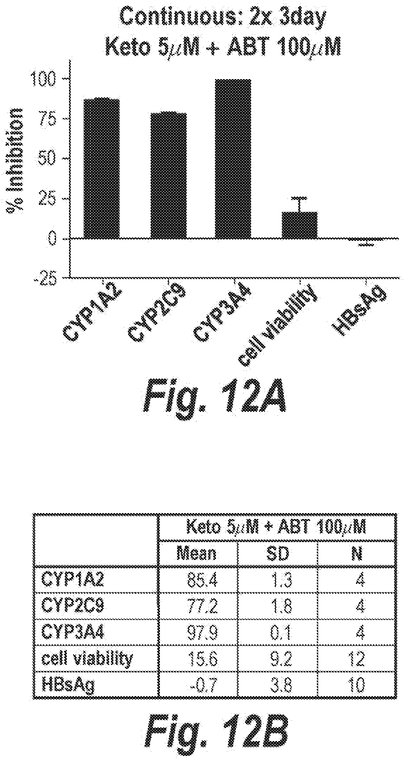

Figures 12A - 12B shows a graph (Fig. 12A) of the data found in the table of Fig. 12B of continuous CYP inhibitor treatment with the CYP3A4-selective inhibitor ketoconazole (5 µM) in combination with ABT (100 µM) of HBV-infected HepaRG cells. The CYP inhibitors were applied on days 5 and 8 p.i., and the assay endpoints measured at day 11 p.i.

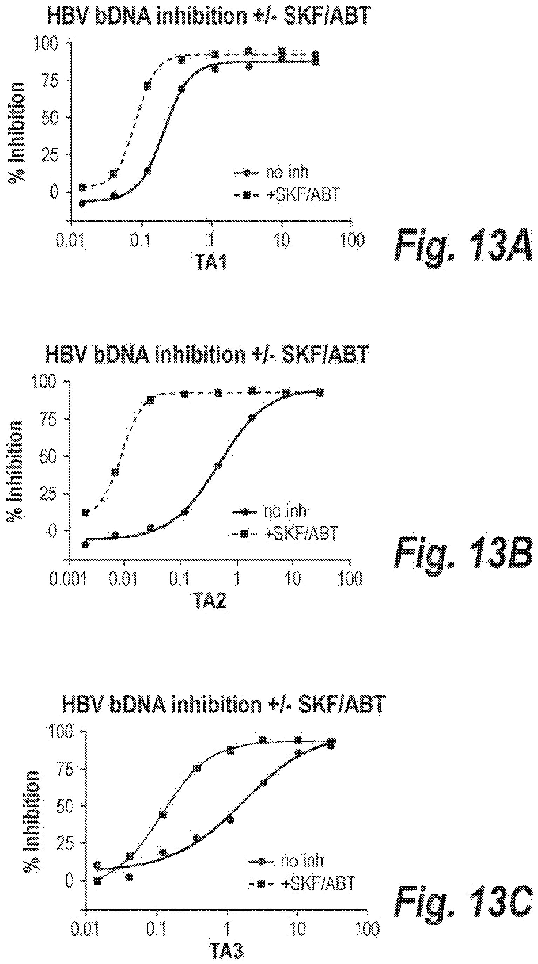

Figure 13A - 13C shows graphs of the results of the antiviral activity of three test compounds (TA1, TA2, and TA3 at the indicated concentrations (in µM)) on the presence of HBV DNA when HBV-infected HepaRG cells are treated or untreated with the combination of 50 µM SKF-525A and 10 mM ABT, wherein the cells are treated with the CYP inhibitors for 1 hour followed by addition of test compound on days 5 and 8 p.i., test compounds were incubated with cells for a total of 6 days, and the assay endpoints measured on day 11 p.i.

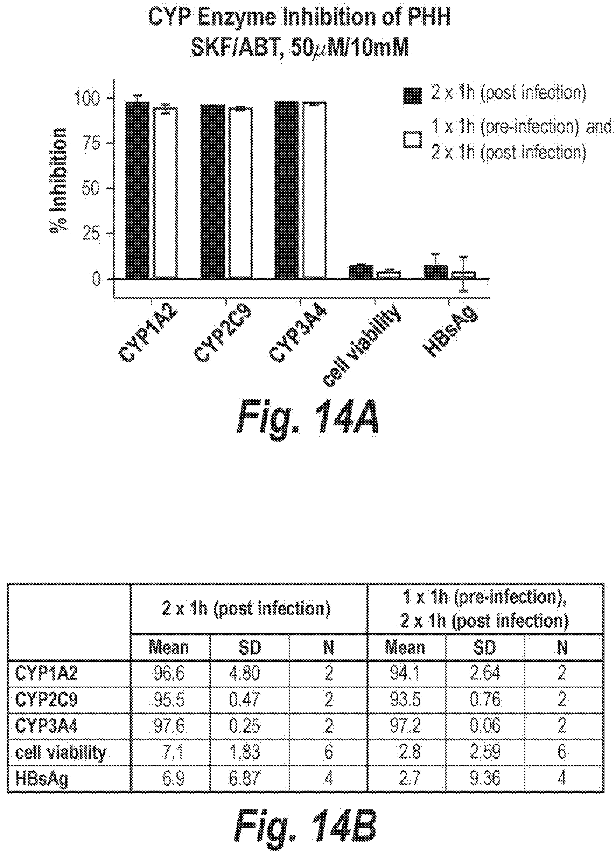

Figures 14A - 4B show a graph (Fig. 14A) of the data found in the table of Fig. 14B presenting the results of cryopreserved primary human hepatocytes (PHH donor 4038) treated with SKF-525A and ABT at the concentrations indicated. In one condition, hepatocytes were first infected with HBV and then treated with CYP inhibitors for one hour on day 5 and day 8 post infection. In another condition, hepatocytes were first treated for one hour with CYP inhibitors prior to HBV infection, followed by treatment of CYP inhibitors for one hour on day 5 and day 8 post infection. The assay endpoints were measured on day 11 p.i.

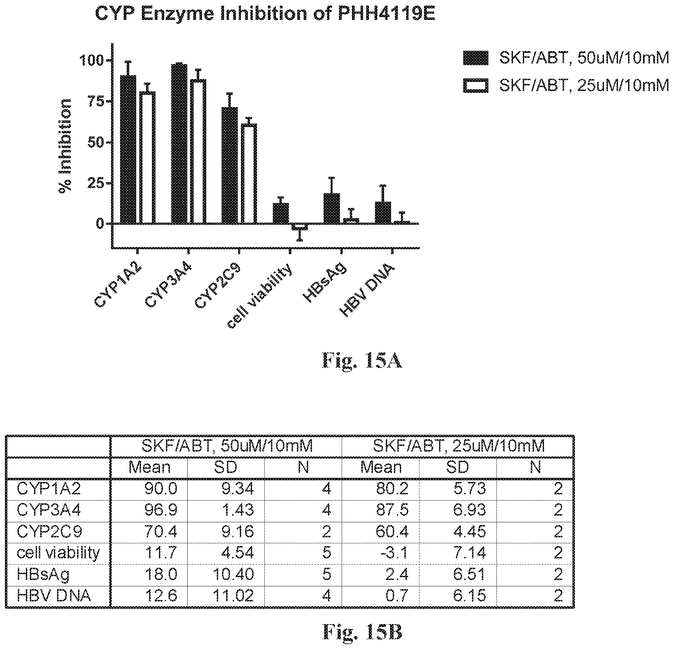

Figures 15A - 15B show a graph (Fig. 15A) of the data found in the table of Fig. 15B presenting the results of cryopreserved primary human hepatocytes (PHH donor 4119E) treated with SKF-525A and ABT at the concentrations indicated. In one condition, hepatocytes were first infected with HBV and then treated with CYP inhibitors (ABT at 10 mM and SKF-525A at 0.05 mM) for one hour on day 5 and day 8 post infection. In another condition, hepatocytes were first infected with HBV and then treated with CYP inhibitors (ABT at 10 mM and SKF-525A at 0.025 mM) for one hour on day 5 and day 8 post infection. The assay endpoints were measured on day 11 p.i.

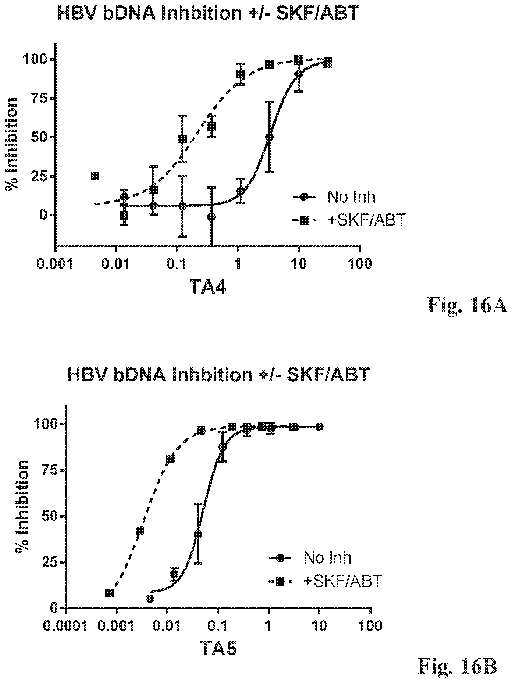

Figures 16A - 16B each show graphs of the results of the antiviral activity of two test compounds (TA4 and TA5 at the indicated concentrations (in µM)) on the presence of HBV DNA when HBV-infected PHH (donor 4119E) are treated or untreated with the combination of 50 µM SKF-525A and 10 mM ABT, wherein the cells are treated with the CYP inhibitors for 1 hour followed by addition of test compound on days 5 and 8 p.i., test compounds were incubated with cells for a total of 6 days, and the assay endpoints measured on day 11 p.i.

### DETAILED DESCRIPTION

### I. Metabolic restriction in cell-based assays

### Liver-related disease agent assays

The ability to modify the metabolic activity of cells while maintaining cell viability, particularly in primary human hepatocytes (PHH), allows for the development of treatments for diseases of or related to the liver. Examples of diseases of, or related to, the liver include malaria, diabetes, viral hepatitis, such as, for example, HBV infection, nonalcoholic steatohepatitis, fatty liver disease, and cirrhosis. As such, provided herein are methods and assays for identifying and optimizing test agents for the treatment of liver-related disease. Test agents used for treatment of liver-related disease can be susceptible to being metabolized by hepatocytes under standard assay conditions; by modulating (e.g., inhibiting) the metabolic activity of the cell, the test agents are minimally metabolized or not metabolized, and the effect of the test agent on the function of the cell can be detected and measured without interference by metabolites of the test agent.

Thus, in one aspect, disclosed herein are methods of identifying test agents to treat liver-related disease in an assay, wherein the assay comprises applying a test agent to primary human hepatocytes, wherein the metabolic capacity of the PHH is or will be modulated such that the test agent is not metabolized or is minimally metabolized during the assay, comprising the steps of, in any order:
(a) treating a cell with one or more enzyme modulators that modulate the metabolic capacity of the cell; and
(b) applying the test agent to the PHH.

Accordingly, the present disclosure describes a method of suppressing metabolism of test agents applied to a treated hepatocyte.

In an aspect, the method comprises
(i) treating an hepatocyte to form a treated hepatocyte;
(ii) adding one or more CYP inhibitors to a buffer to form a CYP inhibitor solution;
(iii) diluting the CYP inhibitor solution with cell growth media to form CYP inhibitor-containing growth media;
(iv) adding the treated hepatocyte to the CYP inhibitor-containing growth media;
(v) incubating the CYP inhibitors with the treated hepatocyte to prepare a CYP-inhibited hepatocyte;
(vi) removing the growth media containing the CYP-inhibitors from the CYP-inhibited hepatocyte;
(vii) adding one or more test agents to the CYP-inhibited hepatocyte;
(viii) incubating the test agent with the CYP-inhibited hepatocyte to form an agent-treated hepatocyte;
(ix) removing the test agent from the agent-treated hepatocyte; and
(x) conducting HBV analytical assays on agent-treated hepatocyte.

As used above, the phrase "treating a hepatocyte" can refer to infecting a hepatocyte with a pathogen such as a parasite, bacterium, or virus, such as Hepatitis B virus.

In the second step of the above method, the CYP inhibitors can be classified as selective inhibitors or non-selective inhibitors of CYP. For example, the CYP inhibitors can be proadifen hydrochloride (SKF-525A) and 1-aminobenzotriazole.

In the second, third, and fourth steps of the above method, the CYP inhibitors can be prepared in a buffer, such as a polar aprotic solvent. A non-limiting example of such a solvent is dimethyl sulfoxide (DMSO).

In the fourth step of the above method, the concentration of CYP inhibitors can range from about 2 µM to about 100 µM, about 5 mM to about 20 mM, about 2 µM or 50 µM, about 0.1 mM, about 0.2 mM or about 10 mM.

In the seventh step of the above method, the test agent can be diluted in phosphate-buffered saline, or growth media such as HepaRG growth media.

In the first and eighth steps of the above method, the hepatocyte can be incubated in growth media containing a test agent for a suitable period of time such that the test agent, if possessing antiviral activity, inhibits a viral activity. For example, test agents can be incubated with the cells for short periods of time, such as about 1 hour to about 8 hours. Furthermore, test agents can be incubated with the cells for days, such as about 1, 2, 3, 4, or 5 days.

In embodiments of the assays disclosed herein, cells are infected with a pathogen, such as *Plasmodium.* In disclosed embodiments, at least one of five species of *Plasmodium* selected from the group consisting of *Plasmodium falciparum, vivax, ovale, malariae, and knowlesi* are used to infect PHH. In disclosed embodiments, at least one of the four species of *Plasmodium* that have been described in African murine rodents selected from the group consisting of *Plasmodium berghei, chabaudi, vinckei, and yoelii* are used. In disclosed embodiments, the viability of the modulated cell and the ability to achieve an endpoint is maintained in the presence of pathogenic infection and enzyme modulation. In disclosed embodiments, an assay endpoint is an event that can be measured, including maturation of sporozoites. In disclosed embodiments, the metabolic capacity of the PHH is modulated before applying the test agent. In disclosed embodiments, the one or more enzyme modulators comprise cytochrome P450 inhibitors.

In yet another aspect, disclosed herein are assays for identifying a malaria treatment or agents for treatment of malaria, such as inhibitors of *Plasmodium* sporozoite maturation, wherein the assay comprises a PHH infected with *Plasmodium,* wherein the metabolic capacity of the *Plasmodium-*infected cell is or will be modulated, and applying a test agent to the *Plasmodium-*infected cell such that the test agent is not metabolized or is minimally metabolized during the assay. In an embodiment, the metabolic capacity of the *Plasmodium-*infected cell is modulated after infecting the cell with *Plasmodium.* In another embodiment, the metabolic capacity of the *Plasmodium-infected* cell is modulated before applying the test agent. In an embodiment, the *Plasmodium-*infected cell is treated with one or more enzyme modulators that modulate the metabolic capacity of the *Plasmodium-infected* cell. In another embodiment, the viability of the modulated cell is maintained. In an embodiment, an assay endpoint is maintained. In yet other embodiments, the modulation of the cell metabolic capacity is accomplished by contacting the cell with one or more cytochrome P450 inhibitors. Non-limiting examples of cytochrome P450 inhibitors that can be used in the assay include SKF-525A and 1-aminobenzotriazole.

In another aspect, disclosed herein are methods of identifying inhibitors of *Plasmodium* sporozoite maturation in an assay, wherein the assay comprises applying a test agent to a *Plasmodium-*infected cell, wherein the *Plasmodium-infected* cell is treated with one or more cytochrome P450 inhibitors that modulate or inhibit the metabolic capacity of the *Plasmodium-*infected cell such that the test agent is not metabolized or is minimally metabolized during the assay, and wherein cell viability and an assay endpoint is maintained.

Also provided herein are assays for identifying treatment agents for Type 2 diabetes, such as inhibitors of mitochondrial respiratory chain complex I in an assay. In an embodiment, the assay comprises a PHH, wherein the metabolic capacity of the PHH is or will be modulated, and applying a test agent to the PHH such that the test agent is not metabolized or is minimally metabolized during the assay. In disclosed embodiments, the metabolic capacity of the PHH is modulated after application of the test agent. In disclosed embodiments, the metabolic capacity of the PHH is modulated before the application of the test agent. In disclosed embodiments, the PHH cell is treated with one or more enzyme modulators that modulate the metabolic capacity of the PHH. In disclosed embodiments, the viability of the modulated cell is maintained. In disclosed embodiments, an assay endpoint is maintained. In disclosed embodiments, an assay endpoint is an event that can be measured, including hepatic glucose production and an increase in AMP-activated protein kinase activity. In disclosed embodiments, the modulation of the cell metabolic capacity is accomplished by contacting the cell with one or more cytochrome P450 inhibitors. Non-limiting examples of cytochrome P450 inhibitors that can be used in the assay include SKF-525A and 1-aminobenzotriazole.

In another aspect, disclosed herein are methods of identifying inhibitors of mitochondrial respiratory chain complex I in an assay, wherein the assay comprises applying a therapeutic test agent to a PHH, wherein the PHH is treated with one or more cytochrome P450 inhibitors that modulate or inhibit the metabolic capacity of the PHH such that the therapeutic test agent is not metabolized or is minimally metabolized during the assay, and wherein cell viability and an assay endpoint is maintained.

In certain embodiments of the assays disclosed herein, the concentration of the cytochrome P450 enzyme inhibitor is about 2 µM to about 100 µM, about 5 mM to about 20 mM, about 2 µM or 50 µM, about 0.1 mM, about 0.2 mM or about 10 mM.

The methods disclosed herein enable the direct detection of test agents in an assay using cells with a modulated metabolic capacity while the cell viability and assay endpoints are maintained. An assay endpoint is a point during the assay where parasite maturation, hepatic glucose production, or cell viability is measured. In embodiments of the methods of these aspects, the assay endpoint can be an event in the life-cycle of the parasite that can be measured, such as sporozoite maturation. In other embodiments, the assay endpoint is a decrease in hepatic glucose production. In other embodiments, the assay endpoint is an increase in AMP-activated protein kinase activity. In other embodiments, the assay endpoint is cell viability.

In other embodiments, the test agent is identified as an antiparasitic agent and inhibitor of *Plasmodium* sporozoite maturation when the appropriate assay endpoints are compared to a control cell in the assay lacking the test agent. In another embodiment, the test agent is identified as an antidiabetic agent and inhibitor of mitochondrial respiratory chain complex I inhibitor when the appropriate assay endpoints are compared to a control cell in the assay lacking the test agent.

In the aforementioned embodiments, the cell can be human, a liver cell, a differentiated liver cell, and a cell from a cell line or a primary culture cell. In embodiments wherein the cell is from a cell line, it can be a HepaRG cell; in those embodiments wherein the cell is a primary culture cell, the cell can be a primary hepatocyte, such as a primary human hepatocyte or a cryopreserved primary human hepatocyte. In embodiments wherein the cell is from an inducible pluripotent stem cell differentiated into hepatocyte-like cell, the cell can be an induced pluripotent stem cell-derived hepatocyte.

### HBV antiviral agent assays

In particular, the assays disclosed herein are useful for identifying and optimizing test agents for the treatment of HBV infection. It has been discovered that cells either infected or uninfected with HBV can metabolize test agents leading to erroneous assay results. In addition, inhibition of the CYP metabolic capacity of those cells preserves test agent and provides an authentic test result. Thus, the test agent (i.e., a substance, such as a compound, being assayed for an activity, such as antiviral activity) is either partially or fully converted to a metabolic mixture presenting an obstacle to identifying authentic hits (i.e., a test agent that appears to have a desired activity, such as anti-HBV activity).

To prevent this metabolic destruction of the parent test agents, but to continue use of the metabolically active cells, it has been discovered that metabolic inhibitors can be added to the cell-based HBV assay and that those inhibitors significantly block the metabolic destruction of the test agents allowing the recognition of different test agents as authentic hits in the assay. This approach was possible because HBV replication and cell viability were not significantly inhibited in cells treated as described with those metabolic inhibitors. Without inhibiting the metabolism of the test agent, the decision about activity of the parent test agent was often incorrectly made because of false positive and false negative results. The disclosed methods enable the antiviral activity of a test agent to be directly measured instead of observing the effect of a pool of metabolites generated from the test agent. In this way, the effects of test agents that are HBV antiviral agents are not confounded by the effects of metabolites of the test agents. The system is robust, supports screening of chemical libraries, and can be used to identify and optimize lead test agents.

Provided herein are methods that allow for identifying test agents as HBV antiviral agents in assays that use metabolically active cells, including HBV-infected cells, such that the activity of these test agents on HBV infection is directly detected. The methods enable such direct detection of the antiviral effects of test agents because the metabolic capacity of the cells in the assays is modulated or inhibited while maintaining cell viability and HBV infection endpoints (an endpoint is a stage in the HBV life cycle that can be detected and/or measured). That is, HBV can complete its life-cycle in such metabolically-modulated cells during the time of the assay.

The methods comprise modulating (e.g., inhibiting) the metabolic capacity of a cell, infecting the cell with HBV, applying a test agent to the cell (these three steps can be done in any order), and then determining if an HBV infection endpoint is altered. In some embodiments, detection is optimized when the metabolic capacity of the cell is modulated or inhibited a second time. In further embodiments, the test agent is also applied to the cell a second time before determining if an HBV infection endpoint is reduced or eliminated. In some cases, metabolic capacity is modulated directly, such as by using inhibitors of cytochrome P450 enzymes. In certain embodiments, the concentration of the cytochrome P450 enzyme inhibitor is about 2 µM to about 100 µM, about 5 mM to about 20 mM, about 2 µM or 50 µM, about 0.1 mM, about 0.2 mM or about 10 mM.

Even though the metabolism of the cells is modulated or inhibited at some point during the assay, the infection of such cells with HBV (and in the absence of a test agent) still results in the nuclear import of HBV protein and DNA, the formation of cccDNA in the nucleus of such cells and the production of HBV RNA from the so-formed cccDNA. The production of HBV RNA in turn results in the downstream production of HBV gene products, including HBV proteins, HBV capsids, infectious HBV particles and subviral particles. The methods therefore are suitable models of natural HBV infection, persistent infection and natural, cccDNA-dependent HBV replication.

Thus, provided herein are methods of identifying a hepatitis B virus (HBV) antiviral agent in an assay, wherein the assay comprises an HBV-infected cell, wherein the metabolic capacity of the HBV-infected cell is or will be modulated or inhibited, and applying a test agent to the HBV-infected cell such that the test agent is not metabolized or is minimally metabolized during the assay.

In one aspect, disclosed herein is a method of identifying a hepatitis B virus (HBV) antiviral agent comprising:
(i) treating a metabolizing cell with one or more enzyme modulators;
(ii) infecting the metabolizing cell with HBV;
(iii) applying a test agent to the HBV-infected cell; and
(iv) determining if the test agent achieves an HBV endpoint, wherein achievement of the HBV endpoint identifies the test agent as an HBV antiviral agent.

In another aspect, the method comprises
(i) combining proadifen hydrochloride (SKF-525A) and 1-aminobenzotriazole (ABT) in a solvent;
(ii) diluting the solution of step (i) in HepaRG growth media;
(iii) adding the diluted ABT and SKF-525A from step (ii) to the HBV-infected cells post infection;
(iv) incubating the CYP inhibitors with HBV-infected cells to prepare CYP-inhibited cells;
(v) removing the growth media containing SKF-525A and ABT from CYP-inhibited cells;
(vi) diluting a test agent in DMSO into HepaRG growth media and adding the diluted test agent in media to CYP-inhibited cells from step (v);
(vii) incubating the test agent with CYP-inhibited cells;
(viii) removing the test agent from CYP-inhibited cells;
(ix) incubating the CYP inhibitors with HBV-infected cells to prepare CYP-inhibited cells;
(x) removing the growth media containing SKF-525A and ABT from the CYP-inhibited cells;
(xi) adding the test agent in DMSO to CYP-inhibited cells;
(xii) incubating the test agent with CYP-inhibited cells to produce the agent-treated cells;
(xiii) removing the test agent from the agent-treated cells; and
(xiv) conducting HBV analytical assays on the agent-treated cells.

In another aspect, the method comprises
(i) combining proadifen hydrochloride (SKF-525A) and 1-aminobenzotriazole (ABT) in DMSO;
(ii) diluting the solution of step (i) at a ratio of 1:50 in HepaRG growth media to achieve a final concentration of 10 mM ABT and 50 µM SKF-525A in 2% DMSO;
(iii) adding the diluted ABT and SKF-525A from step (ii) to the HBV-infected cells on day 5 post infection;
(iv) incubating the CYP inhibitors with HBV-infected cells for 60 minutes at 37°C and 5% CO₂ to prepare CYP-inhibited cells;
(v) removing the growth media containing SKF-525A and ABT from CYP-inhibited cells;
(vi) diluting test agents in DMSO at a ratio of 1:50 into HepaRG growth media and adding the diluted test agents in 2% DMSO media to CYP-inhibited cells from step (v);
(vii) incubating the test agents with CYP-inhibited cells for 3 days;
(viii) removing the test agents from CYP-inhibited cells;
(ix) incubating the CYP inhibitors with HBV-infected cells for 60 minutes at 37°C and 5% CO₂ to prepare CYP-inhibited cells;
(x) removing the growth media containing SKF-525A and ABT from the CYP-inhibited cells;
(xi) adding the test agents in DMSO at a ratio of 1:50 to CYP-inhibited cells;
(xii) incubating the test agents with CYP-inhibited cells for 3 days to produce the agent-treated cells;
(xiii) removing the test agents from the agent-treated cells; and
(xiv) conducting HBV analytical assays on the agent-treated cells.

In yet another aspect, the method comprises
(i) combining proadifen hydrochloride (SKF-525A) and 1-aminobenzotriazole (ABT) in DMSO;
(ii) diluting the solution of step (i) at a ratio of 1:50 in PHH growth media to achieve a final concentration of 10 mM ABT and 50 µM SKF-525A, or 10 mM ABT and 25 µM SKF-525A in 2% DMSO;
(iii) adding the diluted ABT and SKF-525A from step (ii) to PHH at day 0 pre-infection and incubate for 1 hour;
(iv) removing media containing ABT and SKF-525a from step (iii)
(v) adding PHH growth media containing HBV inoculum and 1:50 diluted test agents in DMSO to CYP-inhibited cells from step (iv) and allowed to incubate overnight;
(vi) removing media containing HBV and test agents after the overnight incubation (day 1 post infection), cells were washed 3 times
(vii) incubating the CYP inhibitors with HBV-infected cells for 60 minutes at 37°C and 5% CO₂ to prepare CYP-inhibited cells;
(viii) removing the growth media containing SKF-525A and ABT from CYP-inhibited cells;
(ix) diluting test agents in DMSO at a ratio of 1:50 into PHH growth media and adding the_diluted test agents in 2% DMSO media to CYP-inhibited cells from step (v);
(x) incubating the test agents with CYP-inhibited cells for 3 days;
(xi) removing the test agents from CYP-inhibited cells;
(xii) incubating the CYP inhibitors with HBV-infected cells for 60 minutes at 37°C and 5% CO₂ to prepare CYP-inhibited cells;
(xiii) removing the growth media containing SKF-525A and ABT from the CYP-inhibited cells;
(xiv) adding the test agents in DMSO at a ratio of 1:50 to CYP-inhibited cells;
(xv) incubating the test agents with CYP-inhibited cells for 3 days to produce the agent-treated cells;
(xvi) removing the test agents from the agent-treated cells; and
(xvii) conducting HBV analytical assays on the agent-treated cells.

Also disclosed herein are methods of identifying hepatitis B virus (HBV) antiviral agents in an assay, wherein the assay comprises applying a test agent to an HBV-infected cell, wherein the metabolic capacity of the HBV-infected cell is or will be modulated such that the test agent is not metabolized or is minimally metabolized during the assay, comprising the steps of, in any order:
(a) treating a cell with one or more enzyme modulators that modulate the metabolic capacity of the cell
(b) infecting the cell with HBV; and
(c) applying the test agent to the HBV-infected cell.

Furthermore, disclosed herein are methods of identifying hepatitis B virus (HBV) antiviral agents in an assay, wherein the assay comprises applying a test agent to an HBV-infected cell, wherein the HBV-infected cell is treated with one or more cytochrome P450 inhibitors that modulate or inhibit the metabolic capacity of the HBV-infected cell such that the test agent is not metabolized or is minimally metabolized during the assay, and wherein cell viability and an assay endpoint is maintained.

While not wishing to be bound to any particular theory, it is believed that the use of enzyme modulators or inhibitors of metabolic enzymes that modulate or inhibit the metabolic capacity of cells allows for increased sensitivity of the disclosed assays, enabling discrimination of test agents having antiviral activity, such as those test agents that are cccDNA-inhibitors or HBV replication inhibitors. It has been discovered that without the use of these enzyme modulators or inhibitors, test agents are metabolized during the assay and test results are those of a metabolic mixture and not that of the parent test agent. The use of such enzyme modulators or inhibitors in these assays allows for the detection and prioritization of lead test agents for the discovery of HBV antiviral agents.

In the disclosed methods and assays, the viability of the cell is maintained during the method even though its metabolic capacity is modulated or inhibited, as are assay viral endpoints, which can be, for example, antiviral innate immune genes expression and/or protein production, antiviral genes expression and/or protein production, HBV cccDNA, degraded HBV cccDNA, transcription of HBV cccDNA, nuclear import of HBV polypeptides or HBV DNA, nuclear export of HBV polypeptides or HBV DNA, a function of HBV polypeptide, a function of HBV core polypeptide, HBV capsid polypeptide assembly, HBV replication, HBV genome encapsidation, HBV genome replication, HBV RNA encapsidation, HBV RNA secretion, HBV RNA splicing, HBV virion morphogenesis, HBV virion secretion, the presence of HBsAg antigen, and the presence of HBeAg antigen. In the disclosed assays, a test agent can be identified as an HBV antiviral agent when the appropriate HBV assay endpoints are compared to control cells in the same assay, but lacking the test agent, wherein an endpoint is diminished or absent when compared to the control.

In an embodiment, test agents that are identified by the disclosed methods and in the assays are useful for treating and preventing HBV infection in human subjects. The identified antiviral agents can be optimized to exhibit favorable metabolic, tissue distribution, safety and pharmaceutical profiles, to become suitable for use in human subjects. HBV antiviral agents can, for example, modulate or disrupt HBV replication, HBV core protein function, cccDNA function, or decrease cccDNA levels by interaction with HBV or human proteins or nucleic acids, which can be measured as a reduction of HBV RNA and HBV DNA production. In some embodiments, these identified antiviral agents can, for example, modulate or disrupt cccDNA transcriptional activity, cccDNA levels, formation of cccDNA, degradation of cccDNA, nuclear import or export of HBV proteins or DNA, HBV protein function, HBV assembly and other HBV core protein functions necessary for the generation of infectious particles by interacting with HBV proteins, core protein, capsid, HBV polymerase or human proteins or nucleic acids, to afford defective viral particles with greatly reduced virulence. Furthermore, these identified antiviral agents can induce antiviral protein expression in HBV-infected cells.

In some embodiments, the HBV antiviral agents identified by the disclosed methods and assays are HBV replication inhibitors. These antiviral agents can target the HBV core protein that plays essential functions during the viral life cycle. HBV capsid/core proteins form metastable viral particles or protein shells that protect the viral genome during intercellular passage, and also play a central role in the viral replication processes, including genome encapsidation, genome replication, nuclear import of HBV DNA, cccDNA formation, cccDNA function, HBV RNA splicing, HBV virion morphogenesis and HBV virion secretion. Capsid structures also respond to environmental cues to allow un-coating after viral entry. Consistently, proper capsid assembly and function of core protein have been found to be critical for viral infectivity and cccDNA formation. HBV cccDNA is essential for HBV replication in infected cells as the source of full-length HBV RNA and HBV mRNAs. The clearance of cccDNA is considered the optimal pathway towards achieving an effective HBV treatment that eradicates the virus.

Thus, the HBV antiviral agents identified by the disclosed methods and assays can be useful in HBV treatment by a number of mechanisms, such as by disrupting, accelerating, reducing, delaying and/or inhibiting cccDNA formation, cccDNA function, normal viral capsid assembly and/or disassembly of immature or mature particles, thereby leading to antiviral effects such as reduced cccDNA formation, loss of cccDNA activity (HBV RNA synthesis), loss of cccDNA, disruption of virion assembly and/or disassembly, virion maturation, and/or virus egress.

In one embodiment, the identified HBV antiviral agents disrupt and/or accelerate capsid assembly and/or disassembly when the capsid protein is immature. In another embodiment, the identified HBV antiviral agents disrupt and/or accelerate capsid assembly and/or disassembly when the capsid protein is mature. In yet another embodiment, the identified HBV antiviral agents disrupt and/or accelerate capsid assembly and/or disassembly during viral infectivity. In yet another embodiment, the disruption and/or acceleration of capsid assembly and/or disassembly attenuates HBV viral infectivity and/or reduces viral load. In yet another embodiment, disruption, acceleration, inhibition, delay and/or reduction of capsid assembly and/or disassembly eradicates the virus from the host organism. In yet another embodiment, eradication of the HBV from a subject advantageously obviates the need for chronic long-term therapy and/or reduces the duration of long-term therapy.

In one embodiment, the identified HBV antiviral agents are suitable for monotherapy and are effective against natural or native HBV strains and against HBV strains resistant to currently known drugs. In another embodiment, the identified HBV antiviral agents are suitable for use in combination therapy.

In another embodiment, the identified HBV antiviral agents can be used in methods of inhibiting or disrupting the activity, stability, function, and viral replication properties of HBV cccDNA. In yet another embodiment, the compounds of the invention can be used in methods of diminishing or preventing the formation of HBV cccDNA.

### II. Definitions

It is noted here that as used in this specification and the appended claims, the singular forms "a", "an", and "the" also include plural reference, unless the context clearly dictates otherwise.

The term "about" or "approximately" means within 10%, and more preferably within 5% (or 1% or less) of a given value or range.

As used herein, "antiviral agent" means an agent that inhibits at least one activity of a virus. For example, in the case of HBV infection, an antiviral agent can stimulate antiviral innate immune gene expression and/or protein production, antiviral gene expression and/or protein production, reduce HBV cccDNA, degrade HBV cccDNA, silence transcription of HBV cccDNA, modify nuclear import of HBV polypeptides or HBV DNA, modify nuclear export of HBV polypeptides or HBV DNA, alter a function of HBV polypeptide, alter a function of HBV core polypeptide, alter HBV capsid polypeptide assembly, alter HBV replication, alter HBV genome encapsidation, alter HBV genome replication, alter HBV RNA encapsidation, alter HBV RNA secretion, alter HBV RNA splicing, alter HBV virion morphogenesis, alter HBV virion secretion, alter the presence of HBsAg antigen, and alter the presence of HBeAg antigen.

As used herein "metabolic capacity" means the ability of a cell to perform metabolic processes. These processes are enzyme-mediated chemical reactions that either build-up (anabolism) or break down (catabolism) molecules, which molecules can be used for storage, immediate use, or as an intermediate in another reaction. Such molecules can also be simply reaction by-products. Metabolic processes are necessary for cell growth, environmental responses, cell structure maintenance, etc.

As used herein, "modulate" refers to the ability of a compound to increase or decrease the function, or activity, of a molecule involved in metabolism, such as a cytochrome P450 enzyme or a glucuronyl transferase. "Modulation," as used herein in its various forms, is intended to encompass antagonism, agonism, partial antagonism and/or partial agonism of the activity associated with such molecules involved in metabolism.

As used herein, "inhibit" means to decrease an activity, such as the activity of a molecule involved in metabolism, such as a cytochrome P450 enzyme or a glucuronyl transferase.

As used herein, an "enzyme modulator" is a molecule that modulates at least one activity of an enzyme.

As used herein, "test agent" means an agent that is being tested for an activity, such as an antiviral activity, including HBV antiviral activity.

In an embodiment, the test agent is a small molecule. The term "small molecule" refers to a non-peptidic, non-oligomeric organic compound either synthesized in the laboratory or found in nature. Small molecules, as used herein, can refer to compounds that are "natural product-like," however, the term "small molecule" is not limited to "natural product-like" compounds. Rather, a small molecule is typically characterized in that it contains several carbon-carbon bonds, and has a molecular weight of less than 1500, although this characterization is not intended to be limiting for the purposes of the present invention.

As used herein, "metabolized" means that a molecule, such as a test agent, is changed by a metabolic process; for example, the molecule can be broken down into smaller molecules, or added to to create a larger molecule.

As used herein, "minimally metabolized" means that a minority of a population of molecules is subjected to metabolic processes, thus leaving the majority of molecules intact. In an embodiment, the phrase "minimally metabolized" can refer to a population of molecules in which less than 50%, of the population of molecules is metabolized. For example, less than 1%, less than 2%, less than 3%, less than 4%, less than 5%, less than 6%, less than 7%, less than 8%, less than 9%, less than 10%, less than 20%, less than 30%, or less than 40%, of the population of moleuces can be subjected to metabolic processes in the assay disclosed herein.

As used herein, an "assay endpoint" (when applied to an HBV antiviral assay) or "HBV infection endpoint" means an event in the life-cycle of the virus that can be measured, including the presence of HBV cccDNA, degraded HBV cccDNA, transcription of HBV cccDNA, nuclear import of HBV polypeptides or HBV DNA, nuclear export of HBV polypeptides or HBV DNA, a function of HBV polypeptide, a function of HBV core polypeptide, HBV capsid polypeptide assembly, HBV replication, HBV genome encapsidation, HBV genome replication, HBV RNA encapsidation, HBV RNA secretion, HBV RNA splicing, HBV virion morphogenesis, HBV virion secretion, the presence of HBsAg antigen, and the presence of HBeAg antigen. Alternatively, an assay endpoint can be monitoring the presence of live cells.

As used herein, an "assay endpoint" (when applied to liver-related disease assay) means an event that can be measured, including a decrease in hepatic glucose production, an increase in AMP-activated protein kinase activity, maturation of sporozoites, and the release of merozoites. In other embodiments, the assay endpoint is cell viability.

As used herein, "cytochrome P450 inhibitors," "CYP450 inhibitors," or "CYP inhibitors" indicates those molecules that can reduce an activity of a CYP450 enzyme.

As used herein, a "liver cell" is a cell that has one more markers specific to liver cells found *in vivo.* However, a liver cell can be used *in vitro.*

As used herein, a "differentiated liver cell" is a cell that has multiple markers specific to liver cells found *in vivo.* Furthermore, differentiated liver cells also have characteristic morphological features, as found *in vivo.* Differentiated liver cells can be used *in vitro.*

As used herein, "primary hepatocyte" means a hepatocyte that is isolated from liver tissue (such as from a subject), and then grown in culture *in vitro.*

As used herein, a "cell line" is a culture of clonal cells that can be maintained *in vitro* for an extended, if not indefinite, period of time.

### HI. Components of the disclosed methods

In the disclosed assays and methods, the principle components are cells, enzyme modulators/inhibitors, the compound (test agent) being tested, and the assay endpoint being monitored. For the HBV assays disclosed herein, the principle components are cells, HBV, enzyme modulators/inhibitors, the compound (test agent) being tested for antiviral activity, and the assay endpoint (HBV infection stage or cell viability) being monitored. Other components, such as cell culture media, differentiation media, substrates, etc., are understood to support the assay (e.g., cell viability and growth, cell differentiation, etc.) and are within the skill of the skilled artisan.

### Cells

In various embodiments, the cells that are used in the assays are human cells. In other embodiments, the cells are liver cells, including differentiated liver cells; these cells can be human cells or non-human cells. In yet other embodiments, the cells are from a cell line (such as HepaRG™ cell line), primary culture cells, such as primary hepatocytes and cryopreserved primary hepatocytes, which can be human or non-human, or are induced pluripotent stem cell-derived hepatocyte. In some embodiments, the cells comprise a sodium taurocholate co-transporting polypeptide (NTCP) receptor for HBV; in some such embodiments, the NTCP receptor is expressed by molecular biology techniques in the cells. Such cells can be infected with HBV in cell culture.

The HepaRG™ cell line is a preferred human hepatic stem cell line available from Biopredic International (Saint Gregoire, France). These cells can be used undifferentiated, or differentiated in culture using dimethyl sulfoxide (DMSO); in the disclosed methods, differentiated cells are preferable as they are permissive to HBV infection (Gripon, P., et al. 2002. PNAS 99(24):15655-15660) and can be used as a natural infection system to discover test agents with antiviral activities targeting any of the steps within the HBV life cycle, including viral entry, cccDNA production and maintenance, HBV RNA transcription, viral and/or host proteins that are involved in regulating the replication and persistence of HBV. Compared to hepatoma cell lines, differentiated HepaRG cells are more similar to human hepatocytes because they express functional liver enzymes, including transporters and cytochrome P450 (CYP) enzymes and are therefore metabolically active (Aninat, C., et al. 2006. Drug Metabolism and Disposition 34(1):75-83; Guillouzo, A., et al. 2007. Chemico-Biological Interactions 168:66-73).

Other suitable cell lines include human HuS-E/2 (also treated with DMSO; Huang, H.-C., et al. 2012. J. Virol. 86(17):9443-9453) and HepG2 (treated with DMSO or a combination of DMSO and 5-aza-2'-deoxycytidine; Paran, N. et al. 2001. EMBO J. 20:4443-4453). In other embodiments, induced pluripotent stem cells (Si-Tayeb, K., et al. 2010. Hepatology 51:297-305; Touboul, T., et al. 2010. Hepatology 51:1754-1765) can be differentiated into hepatocyte-like cells (HLCs) which can also support HBV infection (Schwartz, R.E., et al. 2014. Biotech. Adv. 32:504-513).

In some embodiments, the cells are primary hepatocytes. Primary hepatocytes are hepatocytes that are harvested from liver tissue and grown in culture. Primary hepatocytes can be obtained from human liver tissue, mouse, rabbit, guinea pig, woodchuck, duck, tree shrew, chimpanzee, and fish livers (reviewed in Witt-Kehati, D., et al. 2016. Viruses 8(1). pii: E21). Primary hepatocytes may be isolated and cultured according to any suitable method known in the art.

In some embodiments, the cells express exogenous NTCP receptor, such as human NTCP receptor, which can permit in some cells HBV infection (reviewed in Witt-Kehati, D., et al. 2016. Viruses 8(1). pii: E21). One of skill in the art, using classic molecular biology techniques, can prepare cells that express exogenous NTCP receptor.

One of skill in the art can select appropriate culture media, temperature, carbon dioxide gas, substrates, and other culture conditions to grow the various cells. For example, the HepaRG™ cells can be cultured in William's E medium and grown on collagen-coated substrates; they can be differentiated according to the methods disclosed by Gripon et al. (2002. PNAS 99(24):15655-15660). Microtiter plates are convenient for handling many samples simultaneously.

### HBV Genotypes

In the disclosed assays and methods, different HBV genotypes can be used. There are eight known HBV genotypes, A through H, wherein subgenotypes are recognized for A, B, C, and F genotypes (Kramvis, A., et al. Vaccine 23(19):2409-2423). Human HBV also infects tree shrew and chimpanzee liver cells (Witt-Kehati, D., et al. 2016. Viruses 8(1). pii: E21). However, as understood by one of skill in the art, in the cases where non-human cells are used, other hepatitis viruses can be used to match the origin of the cells. For example, if woodchuck liver cells are used, then woodchuck hepatitis virus can be used. Likewise, for duck liver cells, duck hepatitis B virus can be used.

### Cytochrome P450 Enzyme Modulators/Inhibitors

To modulate or inhibit the metabolic capacity of the cells in the disclosed methods and assays, metabolism enzyme modulators or inhibitors are used. Suitable modulators or inhibitors are those that, at the used concentrations, (1) impair or inhibit cell metabolism for the duration of the assay, (2) are not significantly cytotoxic to the cells for the duration of their application and the assay, and (3) do not have significant HBV antiviral properties for the duration of the assay.

Suitable enzyme modulators or inhibitors include those that modulate/inhibit cytochrome P450 (CYP) enzymes. Among over 50 known CYP enzymes, CYP enzymes from families 1, 2, or 3 are generally involved in metabolizing the majority of drugs (Zanger, U. 2008. Analytical Bioanalytical Chemistry 292:1093-1108; see also McDonnell, A. M. and Dang, C. H. 2013. J. Adv. Pract. Oncol. 4(4):263-268). Inhibitors of these enzymes and others are shown in Table 1. Accordingly, an embodiment uses one or more CYP inhibitors selected from Table 1.

**TABLE 1**

| Modulators/inhibitors of cytochrome P450 (CYP) enzymes | | | | | | | |
|---|---|---|---|---|---|---|---|
| **CYP1A2** | **CYP2B6** | **CYP2C8** | **CYP2C9** | **CYP2C19** | **CYP2D6** | **CYP2E1** | **CYP3A4** |
| Amiodarone | Thiopeta | Anastrozole | Amiodarone | Cimetidine | Abiraterone | Disulfiram | Amiodarone |
| Atazanavir | Ticlodipine | Ezetimibe (p) | Atazanavir | Citalopram | Amiodarone | | Amprenavir |
| Cimetidine | | Gemfibrozil | Cimetidine | Delavirdine | Asenapine | | Aprepitant |
| Ciprofloxacin | | Montelukast | Clopidogrel | Efavirenz | Buproprion | | Atazanavir |
| Citalopram | | Nicardipine | Cotrimoxazole | Felbamate | Celecoxib | | Boceprevir |
| Clarithromycin | | Sulfinpyrazone | Delavirdine | Fluconazole | Chloroquine | | Cimetidine |
| Diltiazem | | Trimethoprim | Disulfiram | Fluoxetine | Chlorpheniramine | | Ciprofloxacin |
| Enoxacin | | | Efavirenz | Fluvastatin | Chlorpromazine | | Clarithromycin |
| Erythromycin | | | Fenofibrate | Fluvoxamine | Cimetidine | | Cyclosporine |
| Estradiol | | | Fluconazole | Indomethacin | Cinacalcet | | Danazol |
| Fluvoxamine | | | Fluorouracil | Isoniazid | Citalopram | | Delavirdine |
| Interferon | | | Fluoxetine | Ketoconazole | Clemastine | | Diltiazem |
| Isoniazid | | | Fluvastatin | Lansoprazole | Clomipramine | | Efavirenz |
| Ketoconazole | | | Fluvoxamine | Modafinil | Cocaine | | Erythromycin |
| Methoxsalen | | | Gemfibrozil | Omperazole | Darifenacin | | Ethinyl Estradiol |
| Mibefradil | | | Imatinib | Oxcarbazepine | Desipramine | | Ezetimibe (p) |
| Tegaserod | | | Isoniazid | Probenecid | Diphenhydramine | | Fluconazole |
| | | | Itraconazole | Ticlodipine | Doxepin | | Fluoxetine |
| | | | Ketoconazole | Topiramate | Doxorubicin | | Fluvoxamine |
| | | | Leflunomide | | Duloxetine | | Gestodene |
| | | | Lovastatin | | Escitalopram | | Imatinib |
| | | | Methoxsalen | | Febuxostat | | Indinavir |
| | | | Metronidazole | | Fluoxetine | | Isoniazid |
| | | | Mexiletine | | Fluphenazine | | Itraconazole |
| | | | Modafinil | | Halofantrine | | Ketoconazole |
| | | | Nalidixic acid | | Haloperidol | | Methylprednisolone |
| | | | Norethindrone | | Hydroxychloroquine | | Mibefradil |
| | | | Norfloxacin | | Hydroxyzine | | Miconazole |
| | | | Omeprazole | | Imatinib | | Mifepristone |
| | | | Contraceptives | | Levomepromazine | | Nefazodone |
| | | | Paroxetine | | Methadone | | Nelfinavir |
| | | | Phenylbutazone | | Metoclopramide | | Nicardipine |
| | | | Probenecid | | Mibefradil | | Nifedipine |
| | | | Sertraline | | Midodrine | | Norethindrone |
| | | | Sulfamethoxazole | | Moclobemide | | Norfloxacin |
| | | | Sulfaphenazole | | Nefazodone | | Norfluoxetine |
| | | | Sulfonamides | | Norfluoxetine | | Oxiconazole |
| | | | Tacrine | | Paroxetine | | Posaconazole |
| | | | Teniposide | | Perphenazine | | Prednisone |
| | | | Ticlodipine | | Propafenone | | Quinine |
| | | | Tipranavir | | Propoxyphene | | Ranolazine |
| | | | Troleandomycin | | Propranolol | | Ritonavir |
| | | | Voriconazole | | Quinacrine | | Roxithromycin |
| | | | Zafirlukast | | Quinidine | | Saquinavir |
| | | | | | Ranitidine | | Sertraline |
| | | | Zileutin | | Ranolazine | | Telaprevir |
| | | | | | Ritonavir | | Telithromycin |
| | | | | | Sertraline | | Troleandomycin |
| | | | | | Tegaserod | | Verapamil |
| | | | | | Terbinafine | | Voriconazole |
| | | | | | Thioridazine | | Zafirlukast |
| | | | | | Ticlodipine | | Zileutin |
| | | | | | Tipranavir | | |
| | | | | | Tripelennamine | | |

In addition to the modulators/inhibitors shown in Table 1, those modulators/inhibitors that modulate or inhibit multiple CYPs can be used, such as, for example, proadifen hydrochloride (SKF-525A; Franklin, M.R. and L.G. Hathaway. 2008. Drug Metabolism and Disposition 36:2539-2546; Emoto, C. 2003. Drug Metab. Pharmacokin. 18(5):287-295) and 1-aminobenzotriazole (ABT; *Ibid*.).

### Test Agents

Examples of test agents include nucleic acids, carbohydrates, lipids, proteins, peptides, peplidomimetics, small molecules, organic compounds with molecular weights from 200 - 850 amu, and other drugs or agents. In one embodiment, the test agents are small molecule compounds with molecular weights from 200- 850amu. Test agents can be obtained as individual compounds by a wide variety of synthetic methods. Test agents can also be obtained by using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, K.S. 1997, Anticancer Drug Des. 12(3):145-167; U.S. Pat. Nos. 5,738,996; and 5,807,683).

Methods for synthesis of molecular libraries have been described, such as by DeWitt, S.H. et al. 1993. PNAS USA 90(15):6909-6913; Erb, E. et al. 1994. PNAS USA 91(24):11422-11426; Zuckermann, R.N., et al. 1994. J. Med. Chem. 37:26782685; Cho, C.Y., et al. 1993. Science 261(5126):1303-1305; Carrell, T., et al. 1994. Angew. Chem. Int. Ed. Engl. 33(20):2059-2061; Carell, T., et al. 1994. Angew. Chem. Int. Ed. Engl. 33(2):2061-2064; and Gallop, M. A., et al. 1994. J. Med. Chem. 37(9):1233-1251. Alternatively, libraries that can be used to supply test agents can be obtained commercially.

### Assay Endpoints

To determine if a test agent has HBV antiviral activity in the disclosed methods and assays, HBV endpoints are monitored. If an HBV activity or presence, is diminished, absent, or possibly enhanced (when compared to proper controls), the test agent is a candidate for further analysis as an HBV antiviral agent.

Any point in the life cycle of the HBV can be monitored for this purpose, and is referred to as an "assay endpoint." Examples of assay endpoints include the induction of antiviral innate immune genes expression and/or protein production, antiviral gene expression and/or protein production, inhibition of HBV cccDNA, degradation of HBV cccDNA, silencing the transcription of HBV cccDNA, and modification of any of the following: nuclear import of HBV polypeptides or HBV DNA, nuclear export of HBV polypeptides or HBV DNA, a function of HBV polypeptide, a function of HBV core polypeptide, HBV capsid polypeptide assembly, HBV replication, HBV genome encapsidation, HBV genome replication, HBV RNA encapsidation, HBV RNA secretion, HBV RNA splicing, HBV virion morphogenesis, HBV virion secretion, the presence of HBsAg antigen, and the presence of HBeAg antigen

Alternatively, instead of examining a specific point in the life cycle of the HBV, cell viability can also be monitored, as the virus kills cells in culture if its activity is not inhibited. In a preferred embodiment, HBV genomic DNA is measured, along with cell viability and the presence of HBsAg antigen.

### IV. Methods

In an aspect, disclosed are methods identifying a hepatitis B virus (HBV) antiviral agent in an assay, wherein the assay comprises an HBV-infected cell, wherein the metabolic capacity of the HBV-infected cell is or will be modulated, and applying a test agent to the HBV-infected cell such that the test agent is not metabolized or is minimally metabolized during the assay.

Also disclosed are methods of identifying a hepatitis B virus (HBV) antiviral agent in an assay, wherein the assay comprises applying a test agent to an HBV-infected cell, wherein the HBV-infected cell is treated with one or more cytochrome P450 inhibitors that modulate or inhibit the metabolic capacity of the HBV-infected cell such that the test agent is not metabolized or is minimally metabolized during the assay, and wherein cell viability and an assay endpoint is maintained.

Furthermore, disclosed are methods of identifying a hepatitis B virus (HBV) antiviral agent in an assay, wherein the assay comprises applying a test agent to an HBV-infected cell, wherein the metabolic capacity of the HBV-infected cell is or will be modulated such that the test agent is not metabolized or is minimally metabolized during the assay, comprising the steps of, in any order:
(a) treating a cell with one or more enzyme modulators that modulate the metabolic capacity of the cell;
(b) infecting the cell with HBV; and
(c) applying the test agent to the HBV-infected cell.

### Virus infection

Infection of the selected cells can be performed using HBV for which the cells are permissive. For infecting cells permissive to human HBV, the HepG2.2.15 cell line that constitutively expresses genotype D HBV is convenient as a source of virus particles (Sells MA, et al. 1987. PNAS USA. 84:1005-1009). Furthermore, virus particles can be isolated from the sera of transgenic mice that express the HBV genome, such as line Tg[HBV1.3]24-3 (Chen CC, et al. 2007. Gene ther. 14:11-19; see also Huang, H.-C., et al. 2012. J. Virol. 86(17):9443-9453). Virus particles can also be from hepatitis B infected patient serum.

After collecting and quantifying the virus, cells are infected at a suitable concentration of virus particles. For example, 10⁶ differentiated HepaRG cells can be infected with 100 µl of a 4 x 10⁹ HBV genome equivalent per milliliter (Gripon, P. et al. 2002. PNAS 99(24): 15655-15660). In another example, differentiated HepaRG cells can be infected at 100 genome equivalents (ge)/cell. Thus cells can be infected at, ge per cell, from about 1 ge per cell to about 50,000 ge per cell. Cells can also be infected at, ge per cell, about 100 to 1000, such as 100, 125, 150, 175, 200, 250, 300, 350, 400, 450, 500, 550, 600, 650, 700, 750, 800, 850, 900, 950, and 1000. In one embodiment, the cells are differentiated HepaRG cells that are infected at 100 ge with HBV produced by HepG2.2.15 cells.

Cells can be infected with HBV particles at any time during the assay, such as before, after, or concomitantly contacting the cells with enzyme modulators/inhibitors, and before, after, or concomitantly contacting the cells with the test agent. In some embodiments, the cells are infected with HBV for a period of time before contacting the cells with a test agent or a enzyme modulator/inhibitor (if not already applied to the cells before infection). In a preferred embodiment, the cells are infected for five days before contacting the cells with a test agent or a enzyme modulator/inhibitor (if not already applied to the cells before infection). If desired, after a period of time incubating the cells with the virus (e.g., overnight; about 8, 9, 10, to 12 hours), the cells can be washed with fresh media to remove virus particles that have not infected cells.

### Application of enzyme modulators/inhibitors

The enzyme modulators/inhibitors (discussed above) can be added to the assay cells in appropriate cell culture media. Other reagents can be added, such as DMSO. In an embodiment, 2% DMSO is used; but one of skill in the art can determine the optimal concentration and desirability of such additional reagents. As is the case for infecting the cells, the enzyme modulators/inhibitors can be added to the cells before, after, or concomitantly with virus, and before, after, or concomitantly added to the cells with the test agent.

The enzyme modulators/inhibitors can be incubated with the cells for a short period of time, e.g., about 5 to 180 minutes (or more), including 10, 15, 20, 25, 30, 35, 40, 45, 50, 55, 60, 65, 70, 75, 80, 85, 90, 100, 110, 120, 130, 140, 150, 160, 170, and 180 minutes. In other embodiments, the cells are incubated with the enzyme modulators/inhibitors for longer periods of time, such as about 180 minutes to days, such as 1, 2, or 3 days. In a preferred embodiment, the cells are incubated for 60 minutes at five days after viral infection. In yet another preferred embodiment, the cells are incubated for 60 minutes a second time at 8 days post viral infection. One of skill in the art, in view of the specification and examples, can optimize modulator/inhibitor concentrations, the length of time for incubating the cells with the modulators/inhibitors, and when to add the modulators/inhibitors in relation to HBV infection, such that cell viability and HBV activities/life cycle are not inhibited, while modulating (e.g., inhibiting) the cells' metabolic capacity such that the test agent, when contacted and incubated with the cells, is not, or is minimally, metabolized.

The optimal concentrations of these modulators/inhibitors can be determined by one of skill in the art. Factors to consider are the degree of modulation/inhibition by the modulators/inhibitors on their intended targets, the effects of the modulators/inhibitors on cell viability, effects of the modulators/inhibitors on HBV life cycle, and the length of incubation (e.g., longer incubation times generally require lower doses of the modulators/inhibitors than shorter incubation times). In an embodiment, the concentration of a modulator/inhibitor is about 1 nM to 500 mM. In another embodiment, the concentration of the modulator/inhibitor is about 1 µM to about 200 mM. In other embodiments, the concentration of the modulator/inhibitor is about, in µM, 1, 2, 3, 4, 5, 6, 7, 8, 9, 10, 11,12, 13, 14, 15, 16, 17, 18, 19, 20, 25, 30, 35, 40, 45, 50, 75, 100, 125, 150, 175, 200, 250, 300, 350, 400, 450, 500, 550, 600, 650, 700, 750, 800, 850, 900, 950, 1000, 1.5 x 10³, 2 x 10³, 2.5 x 10³, 3 x 10³, 3.5 x 10³, 4 x 10³, 4.5 x 10³, 5 x 10³, 7.5 x 10³, 10 x 10³, 15 x 10³, 20 x 10³, 25 x 10³, 30 x 10³, 35 x 10³, 40 x 10³, 45 x 10³, 50 x 10³, 55 x 10³, 60 x 10³, 65 x 10³, 70 x 10³, 75 x 10³, 80 x 10³, 85 x 10³, 90 x 10³, 95 x 10³, and about 100 x 10³. The metabolism modulators/inhibitors can be removed, if desired, before performing a next step, such as virus infection and/or contacting the cells with a test agent.

To measure the activity of the different CYP enzymes, CYP assays can be used, such as those that are commercially available from Promega (Madison, WI) (Nonlytic P450-Glo Assays). In an embodiment, the activity of a CYP enzyme is inhibited by at least 50%-100%, such as 50%, 55%, 60%, 65%, 70%, 75%, 80%, 85%, 90%, 95%, and 100%. In some embodiments, CYP enzymes are overall inhibited by at least 50%-100%, such as 50%, 55%, 60%, 65%, 70%, 75%, 80%, 85%, 90%, 95%, and 100%. In a preferred embodiment, CYP enzyme activity is inhibited by about 90%.

In an embodiment, only one modulator/inhibitor is used. In another embodiment, more than one modulator/inhibitor is used, such as 2, 3, 4, or more modulators/inhibitors. When multiple modulators/inhibitors are used, they can be contacted to the cells sequentially or simultaneously, or a combination thereof. In some embodiments, the cells are contacted with the metabolism modulator(s)/inhibitor(s) a second time after a first incubation with the test agent. In embodiments where the cells are contacted with the modulator(s)/inhibitor(s) twice, then the same modulator(s)/inhibitor(s) or different modulator(s)/inhibitor(s) can be used in each step of contacting the cells.

In a preferred embodiment, HepaRG™ cells are used, and the modulators/inhibitors SKF-525A and ABT are used together preferably at about 25 µM to about 50 µM and at about 5 mM to about 10 mM concentrations respectively at five days after HBV infection for about 60 minutes, and then removed from the culture. In another preferred embodiment, these modulators/inhibitors are applied a second time for 60 minutes at eight days post-viral infection, and then removed from the culture.

### Application of test agents

The cells can be contacted with one or more test agents, optionally at various concentrations (preferably at concentrations that do not significantly affect cell viability), according to methods well-known to one of skill in the art. The test agents can be applied in appropriate cell culture media. Other reagents can be added, such as to optimize/maintain cell culture/cell differentiation. Simultaneously, control cells are contacted with the same solution as used for the test agents, but without any test agents. In some embodiments, cells are untreated.

Cells can be contacted with the test agents at any time during the assay, such as before, after, or concomitantly contacting the cells with enzyme modulators/inhibitors, and before, after, or concomitantly infecting the cells with virus. Importantly, the metabolic capacity of the cells must be modulated/inhibited before, during, or soon after applying the test agent, such that the test agent is minimally or not metabolized. In a preferred embodiment, the test agent is applied on the fifth day after viral infection.

The cells can be contacted with the test agents for a suitable period of time such that the test agent, if possessing antiviral activity, inhibits a viral activity. For example, test agents can be incubated with the cells for short periods of time, such as about 1 hour to about 8 hours. Furthermore, test agents can be incubated with the cells for days, such as about 1, 2, 3, 4, or 5 days. In a preferred embodiment, the test agent is incubated with the cells for three days.

In some embodiments, the cells are contacted with the test agent a second time after a first incubation with the test agent and a second application of metabolism modulator(s)/inhibitor(s). The second application of the test agent can be after, concomitantly, or after the contacting of the cells with the metabolism modulator(s)/inhibitor(s). In a preferred embodiment, the cells are contacted with metabolism modulator(s)/inhibitor(s) a second time for about 60 minutes after a first incubation with the test agent (which is removed from the cells before contacting the cells with the metabolism modulator(s)/inhibitor(s)); after removing the metabolism modulator(s)/inhibitor(s), the cells are contacted with the same test agents a second time at the same concentrations as the first incubation for about three days. In a preferred embodiment, this second application of the test agent is done on day eight post-viral infection.

### Monitoring assay endpoints

Assay endpoints can be detected using molecular biology (polymerase chain reaction (PCR) techniques, nucleic acid hybridization (including branched DNA (bDNA) and Quantigene approaches), gel-based detection of HBV DNA, cccDNA, pregeomic RNA, preS/S RNA, encapsidated RNA, spliced RNA, viral proteins, immunofluorescence imaging of viral and host proteins, etc.) and cell biology approaches, e.g., antibody binding of the HBsAg and HBeAg antigen). Some useful protocols can also be found in Hepatitis B and D Protocols: Volume 1: Detection, Genotypes, and Characterization (Methods in Molecular Medicine) 2004. Ed. R.K. Hamatake and J.Y.N. Lau; Human Press, 376 pp. Depending on the endpoint to be measured, one of skill in the art can select whether to analyze culture supernatant (e.g., the presence of virus particles, HBV genome, HBV antigens), cells (cell viability, presence of cccDNA), or both.

Cell viability of HBV-infected cells can be determined by using, for example, commercially available kits and reagents, such as CellTiter-Glo reagent (Promega; Madison, WI), LIVE/DEAD cell viability kits (ThermoFisher Scientific; Waltham, MA), XTT cell viability kits (Cell Signaling Technology; Danvers, MA), EarlyTox cell viability assay kits (Molecular Devices; Sunnyvale, CA) and various cell viability kits from Sigma-Aldrich (St. Louis, MO).

In a preferred embodiment, HBV DNA is measured from virions found in the cell supernatants, lysing the virions after treating the supernatants with DNase (and inactivating DNase before lysis), denaturing the HBV DNA and then detected by a bDNA assay using appropriate HBV nucleic acid probes. The HBV DNA can also be quantified, using techniques well-known in the art or by using commercially available kits, such as the QuantiGene kit from Affymetrix (Santa Clara, CA).

In a preferred embodiment, the levels of the HBsAg antigen are monitored using an anti-HBsAg antibody to detect the antigen. In addition to well-known methods in the art, commercial kits are also available for this purpose, such as HBsAg chemiluminescent immunoassay from Autobio Diagnostics Co. (China).

In a preferred embodiment, cell viability is also measured.

### Correlating results

To identify a test agent that has antiviral activity, one or more assay endpoints are detected or measured, and the resulting values compared to controls. In an embodiment, a test agent can have antiviral activity when HBV DNA is reduced or absent, and/or when the level of HBsAg is reduced or absent, and/or when cell viability is higher when compared to controls. In a preferred embodiment, HBV DNA levels, HBsAg levels, and cell viability are measured for each condition.

EC₅₀ values, effective concentrations that achieve 50% inhibitory effect, can also be determined by methods well-known in the art.

### VI. Additional exemplary embodiments

Some specific embodiments of the invention supported by the examples include the following:
An embodiment is directed to a method of identifying a liver-related disease treatment agent in an assay, wherein the assay comprises applying a test agent to a primary human hepatocyte (PHH), wherein the metabolic capacity of the PHH has been, or will be, modulated, such that the test agent is not metabolized or is minimally metabolized during the assay. In a further embodiment, the absence or diminished presence of liver-related disease progression, or the presence of a live cell, when compared to a control sample in the assay lacking the test agent, indicates that the test agent is a liver-related disease treatment agent. Yet another embodiment is directed to a method of identifying a liver-related disease treatment agent in in an assay wherein the assay comprises applying the test agent to a PHH, wherein the metabolic capacity of the PHH has been, or will be, modulated such that the test agent is not metabolized or is minimally metabolized during the assay, comprising the steps of:
(a) contacting and incubating a cell with a first enzyme modulator that modulates the metabolic capacity of the cell for a period of time,
or
(a) infecting the PHH with a pathogen, and
(b) contacting and incubating the infected PHH with a first enzyme modulator that modulates the metabolic capacity of the HBV-infected cell for a period of time;
(c) contacting and incubating the PHH with the test agent for a period of time; and
(d) determining whether the test agent diminishes liver-related disease activity.

In an embodiment of the embodiments above, the primary human hepatocyte can be infected with a pathogen, such as Hepatitis B virus. In a further embodiment, the metabolic capacity of the primary human hepatocyte can be modulated by incubating the cell with a cytochrome P450 inhibitor, such as SKF-525 or 1-aminobenzotriazole. A combination of more than one cytochrome P450 inhibitor can be used. A test agent can be applied before the metabolic capacity of the primary human hepatocyte is modulated. In one embodiment of the embodiments above, the test agent can be applied after the metabolic capacity of the primary human hepatocyte is modulated. In yet another embodiment of the embodiments above, the test agent is not metabolized during the assay. In yet another embodiment of the above embodiments, the test agent disrupts disease progression of a liver-related disease. In another embodiment of the above embodiments, the test agent is not cytopathic. For purposes of identifying a compound, infected-primary human hepatocytes incubated with a test agent can be compared to a control sample lacking the test agent.

Another embodiment is directed to a method of identifying a hepatitis B virus (HBV) antiviral agent in an assay, wherein the assay comprises applying a test agent to an HBV-infected cell, wherein the metabolic capacity of the HBV-infected cell has been, or will be, modulated, such that the test agent is not metabolized or is minimally metabolized during the assay. In a further embodiment, the absence or diminished presence of HBV or an activity of HBV, or the presence of a live cell, when compared to a control sample in the assay lacking the test agent, indicates that the test agent is an HBV antiviral agent. In a further embodiment, the cell is contacted with an enzyme modulator that modulates the metabolic capacity of the HBV-infected cell.

Another embodiment is directed to a method of identifying a hepatitis B virus (HBV) antiviral agent in an assay, wherein the assay comprises applying a test agent to an HBV-infected cell, wherein the HBV-infected cell is contacted with cytochrome P450 inhibitor that inhibits, or will inhibit, the metabolic capacity of the HBV-infected cell, such that the test agent is not metabolized or is minimally metabolized during the assay. In a further embodiment, the absence or diminished presence of HBV or an activity of HBV, or the presence of a live cell, when compared to a control sample in the assay lacking the test agent, indicates that the test agent is an HBV antiviral agent.

Yet another embodiment is directed to a method of identifying a hepatitis B virus (HBV) antiviral agent in an assay wherein the assay comprises applying a test agent to an HBV-infected cell, wherein the metabolic capacity of the HBV-infected cell has been, or will be, modulated, such that the test agent is not metabolized or is minimally metabolized during the assay, comprising the steps of:
(a) contacting and incubating a cell with a first enzyme modulator that modulates the metabolic capacity of the cell for a period of time, and
(b) infecting the cell with HBV,
or,
(a) infecting the cell with HBV, and
(b) contacting and incubating the HBV-infected cell with a first enzyme modulator that modulates the metabolic capacity of the HBV-infected cell for a period of time;
(c) contacting and incubating the HBV-infected cell with the test agent for a period of time; and
(d) determining whether the test agent diminishes an HBV activity.

In an embodiment, the absence or diminished presence of HBV or an activity of HBV, or the presence of a live cell, when compared to a control sample in the assay lacking the test agent, indicates that the test agent is an HBV antiviral agent. In a further embodiment, this method can further comprise the steps of:
(e) contacting and incubating the HBV-infected cell with a second enzyme modulator that modulates the metabolic capacity of the HBV-infected cell for a period of time after step (c), and
(f) contacting and incubating the HBV-infected cell with the test agent for a period of time.

An embodiment is directed to a method for identifying a hepatitis B virus (HBV) antiviral agent in an assay, wherein the assay comprises applying a test agent to an HBV-infected cell, wherein the metabolic capacity of the HBV-infected cell has been, or will be, modulated, such that the test agent is not metabolized or is minimally metabolized during the assay, comprising:
(a) infecting a cell with HBV, and
(b) contacting and incubating the HBV-infected cell with a first enzyme modulator that modulates the metabolic capacity of the cell for a period of time;
or,
(a) contacting and incubating the HBV-infected cell with a first enzyme modulator that modulates the metabolic capacity of the cell for a period of time, and
(b) infecting a cell with HBV,
   and
(c) contacting and incubating the HBV-infected cell with the test agent for a period of time;
(d) contacting and incubating the HBV-infected cell with a second enzyme modulator for a period of time;
(e) contacting and incubating the HBV-infected cell a second time with the test agent for a period of time; and
(f) determining whether the test agent diminishes an HBV activity.

In some embodiments, the absence or diminished presence of HBV or an activity of HBV, or the presence of a live cell, when compared to a control sample in the assay lacking the test agent indicates that the test agent is an HBV antiviral agent.

An embodiment is directed to a method for identifying a hepatitis B virus (HBV) antiviral agent in an assay, wherein the assay comprises applying a test agent to an HBV-infected cell, wherein the metabolic capacity of the HBV-infected cell has been modulated, such that the test agent is not metabolized or is minimally metabolized during the assay, comprising:
(a) infecting a cell with HBV, and
(b) contacting and incubating the HBV-infected cell with a first enzyme modulator that modulates the metabolic capacity of the cell for a period of time;
or,
(a) contacting and incubating the HBV-infected cell with a first enzyme modulator that modulates the metabolic capacity of the cell for a period of time, and
(b) infecting a cell with HBV,
   and
(c) contacting and incubating the HBV-infected cell with the test agent for a period of time, with the first enzyme modulator;
(d) contacting and incubating the HBV-infected cell with the second enzyme modulator for a period of time;
(e) contacting and incubating the HBV-infected cell a second time with the test agent with the second enzyme modulator for a period of time; and
(f) determining whether the test agent diminishes an HBV activity.

In some embodiments, the absence or diminished presence of HBV or an activity of HBV, or the presence of a live cell, when compared to a control sample in the assay lacking the test agent indicates that the test agent is an HBV antiviral agent.

An embodiment is directed to a method for identifying a hepatitis B virus (HBV) antiviral agent in an assay, wherein the assay comprises applying a test agent to an HBV-infected cell, wherein the metabolic capacity of the HBV-infected cell has been modulated, such that the test agent is not metabolized or is minimally metabolized during the assay, comprising:
(a) infecting a cell with HBV;
(b) contacting and incubating the HBV-infected cell with a first enzyme modulator that modulates the metabolic capacity of the cell for a period of time;
(c) removing the first enzyme modulator; and
(d) contacting and incubating the HBV-infected cell with a second enzyme modulator that modulates the metabolic capacity of the cell for a period of time;
or,
(a) contacting and incubating the HBV-infected cell with a first enzyme modulator that modulates the metabolic capacity of the cell for a period of time;
(b) removing the first enzyme modulator; and
(c) contacting and incubating the HBV-infected cell with a second enzyme modulator that modulates the metabolic capacity of the cell for a period of time;
(d) infecting a cell with HBV,
   and
(e) contacting and incubating the HBV-infected cell with the test agent with the second enzyme modulator for a period of time;
(f) contacting and incubating the HBV-infected cell a second time with a first enzyme modulator that modulates the metabolic capacity of the cell for a period of time;
(g) removing the first enzyme modulator;
(h) contacting and incubating the HBV-infected cell a second time with a second enzyme modulator that modulates the metabolic capacity of the cell for a second period of time;
(i) contacting and incubating the HBV-infected cell a second time with the test agent with the second enzyme modulator for a period of time; and
(j) determining whether the test agent diminishes an HBV activity.

In further embodiments, the absence or diminished presence of HBV or an activity of HBV, or the presence of a live cell, when compared to a control sample in the assay lacking the test agent indicates that the test agent is an HBV antiviral agent.Additional embodiments include those wherein at least one of the first enzyme modulator and (in the case of using multiple enzyme modulators) second enzyme modulator is an enzyme inhibitor that inhibits the metabolic capacity of the HBV-infected cell, such as an enzyme inhibitor that is a cytochrome P450 inhibitor. In some embodiments, the CYP450 inhibitor is SKF-525 or 1-aminobenzotriazole, or any of those that are presented in Table 1. In other further embodiments, the first and second enzyme modulators are the same. In yet other further embodiments, the first enzyme modulator differs from the second enzyme modulator. In embodiments, the cell is contacted and incubated with one modulator to modulate the metabolic capacity of the cell; yet in other embodiments, the cell is contacted and incubated with multiple modulators to modulate the metabolic capacity of the cell. In some embodiments, the cell is contacted and incubated with multiple modulators simultaneously to modulate the metabolic capacity of the cell; in yet other embodiments, the cell is contacted and incubated with multiple modulators sequentially to modulate the metabolic capacity of the cell. In embodiments wherein the cytochrome P450 inhibitors are SKF-525 and 1-aminobenzotriazole, the concentration of SKF-525 is about 2 µM to about 100 µM, and the concentration of 1-aminobenzotriazole is about 5 mM to about 20 mM. In other embodiments, the concentration of SKF-525 is about 2 µM or 50 µM, and the concentration of 1-aminobenzotriazole is about 0.1 mM, about 0.2 mM or about 10 mM.

In further embodiments, the period of time for contacting and incubating the cell with the first enzyme modulator is about 5 minutes to about 120 minutes; in some embodiments, the time is 60 minutes. In some embodiments, the first enzyme modulator is applied on day five after viral infection. In yet other further embodiments, the period of time for contacting and incubating the cell with the test agent of step (c) is about one to about three days; in some embodiments, this time is about three days. In some embodiments, the modulators/inhibitors are removed from the cells before applying the test agent. In some embodiments, the application of the test agent starts on day five post-viral infection. In further embodiments, the period of time for contacting and incubating the cell with the second enzyme modulator is about 5 minutes to about 120 minutes; in specific embodiments, this time is 60 minutes. In some embodiments, the second enzyme modulator is applied on day eight post-viral infection. In yet further embodiments, the period of time for contacting and incubating the cell with the test agent of step (e) is about one to three days; in some embodiments, this time is three days. In some embodiments, the modulators/inhibitors are removed from the cells before applying the test agent. In some embodiments, this second time of applying the test agent to the cell starts on day eight after viral infection.

In embodiments, the cell is a liver cell, such as a differentiated liver cell and can be a cell from a cell line or a primary hepatocyte. In embodiments, the cell is human; such as a cell that is a HepaRG cell. In other embodiments, the cell comprises a sodium taurocholate cotransporting polypeptide (NTCP) receptor for the HBV. In some embodiments, the NTCP is exogenously expressed.

In an embodiment of the assay, the activity of HBV that is detectable in the HBV-infected cell is selected from the group consisting of cccDNA levels, formation of cccDNA, degradation of cccDNA, cccDNA transcriptional activity, nuclear import of HBV polypeptides or DNA, nuclear export of HBV polypeptides or DNA, an HBV polypeptide function, an HBV core polypeptide function, capsid polypeptide assembly, HBV replication, HBV genome encapsidation, HBV genome replication, HBV RNA splicing, HBV virion morphogenesis, and HBV virion secretion, presence of HBsAg, and presence of HBeAg antigen. In an embodiment, an activity of HBV is detectable in the HBV-infected cell in the presence of one or more enzyme modulators.

### EXAMPLES

### Example 1 -Methods

### Example 1.1 - HBV infection

Differentiated HepaRG cells were seeded at 60,000 cells/well in collagen coated plates and allowed to re-differentiate prior to HBV infection. Inoculum was prepared from the supernatants of HepG2.2.15 cells, a stable cell line that constitutively express genotype D HBV. On day 0, differentiated HepaRG cells were incubated with HBV inoculum at 100 genome equivalent/cell and 4% PEG-8000 at 37°C and 5% CO₂ incubator. After overnight incubation, the inoculum was removed and cells were washed 3 times with William's E Medium. Infected HepaRG cells were maintained in differentiation medium until test agent treatment.

### Example 1.2 - P450 CYP enzymatic assays

Nonlytic P450-Glo Assays were performed on the cells of Example 1.1 as described according to manufacturer recommendations (Promega) for the following CYP enzymes as indicated in the Examples below (CYP3A4, CYP2C9, CYP1A1/1B1, CYP1A2, and CYP2B6). Briefly, supernatant was removed from cells at the end of the tested treatment. To evaluate the effect of CYP inhibitors on the metabolism of a test agent, cells were first washed with PBS and then were incubated with CYP P450-Glo Substrate buffer. The corresponding substrate for each cytochrome was diluted in either HepaRG Growth media (for CYP3A4, CYP2C9, and CYP1A1/1B1) or PBS containing 3 mM salicylamide (for CYP1A2 and CYP2B6). Substrate dilutions were prepared as described except for CYP2C9, in which the substrate was diluted 1:10 instead of 1:50. Diluted substrate (50 µL) was added to cells and allowed to incubate for the recommended duration time in 37°C. After incubation, 25 µL of the substrate-containing solution was removed from the cells and transferred to an opaque 96-well plate to be incubated with the Luciferin Detection Reagent. Luminescence was measured using the Victor X4 plate reader (Perkin Elmer) at the following integration times: CYP1A1/1B1, CYP1A2, CYP2B6, and CYP2C9 = 1 second, and CYP3A4 = 0.2 seconds.

### Example 1.3 - HBV antiviral assays

At the end of the indicated treatment, supernatant was removed from the cells and treated with 2 units of Turbo DNase (Invitrogen) at 37°C for 60 minutes, followed by inactivation of the enzyme at 75°C for 15 minutes. Encapsidated HBV DNA was released from the virions and covalently linked HBV polymerase by incubating in lysis buffer (Affymetrix) at 50°C for 40 minutes. HBV DNA was denatured by addition of 0.2 M NaOH and detected by branched DNA (bDNA) assay using HBV DNA probes and the QuantiGene assay kit according to manufacturer recommendation (Affymetrix). HBsAg levels were monitored by using the HBsAg chemiluminescent immunoassay according to manufacturer recommendation (Autobio). The mean background signal from uninfected cells was subtracted from all other samples, and percent inhibition at each test agent concentration was calculated by normalizing to signals from cells treated with 2% DMSO (no test agent control). Cell viability of HBV-infected cells was determined by using CellTiter-Glo reagent according to the manufacturer protocol (Promega). EC₅₀ values, effective concentrations that achieved 50% inhibitory effect, were determined by non-linear fitting using Prism software (Graphpad).

### Example 2-Assay development: CYP-inhibitor titrations using uninfected, differentiated HepaRG cells

### Example 2.1 - CYP enzyme activity in HepaRG cells

Using the P450-Glo assay according to the manufacturer's instructions (Promega), the activities of CYP1A/1B1, CYP2C9, CYP3A, CYP1A2, and CYP286 were analyzed in uninfected, HepaRG cells under standard culture conditions. The results are shown in Table 2.

**TABLE 2**

| | |
|---|---|
| CYP enzyme activity in HepaRG cells | |

| Enzyme | Signal/Noise |
|---|---|
| CYP1A1/1B1 | 1.6 |
| CYP2C9 | 11.5 |
| CYP3A4 | 326.9 |
| CYP1A2 | 30.6 |
| CYP2B6 | 4.6 |

From these results, no detectable activities for CYP1A1/1B1 were observed, with only a slight increase in activity by CYP2B6. However, CYP1A2 and CYP2C9 were observed to have measurable activity, and CYP3A4 was the most active CYP enzyme.

### Example 3- Assay development using infected, differentiated HepaRG cells

In these examples, the effects of the CYP inhibitors ABT and SKF-525A were evaluated for any effects on HBV replication in infected cells.

### Example 3.1 - Methods summary

Differentiated HepaRG cells were treated as shown in Table 3.

**TABLE 3**

| | |
|---|---|
| Summary of methods for Example 3 | |

| Day post-infection (p.i.) | Step(s) |
|---|---|
| 0 | Infect cells with HBV |
| 5 | 1. Incubate cells with or without CYP inhibitors for one hour. ABT concentrations: 5 mM and 10 mM; SKF-525A concentrations: 20 µM and 200 µM. |
| | 2. HBV antiviral agent-1 (HAA-1) added in the absence of CYP inhibitors |
| 11 | Monitor: |
| | (a) presence of secreted HBsAG |
| | (b) CYP3A4 activity |
| | (c) cell viability (using ATP activity as a marker) |

### Example 3.2 - Effect of CYP inhibitors in HBV-infected cells

When HBsAg secretion was measured on day 11 p.i., it was observed that, regardless of the concentrations tested of the CYP inhibitors, HBsAg secretion was observed to be at approximately similar levels (Fig. 2); thus the CYP inhibitors appeared to not affect HBsAg secretion.

### Example 3.3 - Effect of CYP inhibitors on an HAA-1 activity

HAA-1 was added to the infected cells on day 5 p.i. at both 10 µM and 50 µM, and HBsAg secretion and cell viability measured on day 11 p.i. When infected cells are treated with SKF-525A at the concentration that was observed to inhibit CYP3A4 in HBV-infected cells (Figure 5B, 200 µM), HAA-1's antiviral activities were observed to be reduced (Figs. 3A and 3B). Furthermore, when HAA-1 was added to the HBV-infected cells at 50 µM, cell cytotoxicity was observed to be reduced (Fig. 3B). In the absence of SKF-525A, but in the presence of HAA-1, CYP3A4 activity was also observed to be significantly inhibited (Figs. 3A and 3B).

### Example 4- Effect of HBV infection on CYP activities

In this experiment, the effect of HBV infection, if any, on CYP activities was measured. HepaRG cells were untreated, treated with polyethylene glycol (PEG), or infected with HBV, and the activities of CYP2C9, CYP1A2, and CYP3A4 determined eleven days p.i.

As can be seen from the results summarized in Fig. 1, the activity of the CYP enzymes was observed to be detected at similar levels, regardless if the cells were untreated (uninfected), treated with PEG (vehicle, uninfected), or infected with HBV. CYP2C9 was measurable after increasing substrate concentration. As was determined in Example 2, CYP1A2 had measurable activity, and CYP3A4 had high activity.

### Example 5- Assay development: combinations of CYP inhibitors to maximize inhibition of CYP1A2, CYP2C9, and CYP3A 4

### Example 5.1 - Evaluation of CYP1A2. CYP2C9. and CYP3A4 inhibition: ABT

The effects of different ABT treatment regimens on CYP1A2, CYP2C9, and CYP3A4 activity were determined in HBV-infected HepaRG cells. Cell viability and HBsAg secretion were also monitored.

In a first set of experiments, infected cells were treated with 10 mM or 20 mM ABT for one hour applied twice (at days 5 and 8 p.i.). The results (obtained on day 11 p.i.) are shown in the table of Fig. 4B, which data are shown graphically in Fig. 4A. In another set of experiments, infected cells were treated twice for 3 days with 0.1 mM or 0.2 mM ABT at days 5 and 8 p.i. The results of these experiments are presented in the table of Fig. 4D, which data are shown graphically in Fig. 4C. From these experiments, it was observed that ABT inhibited CYP3A4 slightly better than CYP1A2 (>90%), which was greater than inhibition of CYP2C9 (72%-75%).

### Example 5.2 - Evaluation of CYP1A2, CYP2C9, and CYP3A4 inhibition: SKF-525A

In this example, the effects of different SKF-525A treatment regimens on CYP1A2, CYP2C9, and CYP3A4 activity were determined in HBV-infected HepaRG cells. Cell viability and HBsAg secretion were also monitored.

In a first set of experiments, infected cells were treated with 100 µM or 200 µM of SKF-525A for one hour applied twice (at days 5 and 8 p.i.). The results (obtained on day 11 p.i.) are shown in the table of Fig. 5B, which data are shown graphically in Fig. 5A. In another set of experiments, infected cells were treated twice for 3 days with 2 µM or 20 µM SKF-525A (at days 5 and 8 p.i.). The results of these experiments are presented in the table of Fig. 5D, which data are shown graphically in Fig. 5C. From these experiments it was observed that SKF-525A inhibited CYP3A4 better than CYP2C9, and inhibited CYP1A2 less.

### Example 5.3 - Evaluation of sequential treatment with SKF-525A and then ABT

In this experiment, HBV-infected HepaRG cells were treated with SKF-525A for one hour, on day 5 post infection and then with ABT for three days. On day 8 post infection, media containing ABT was removed and cells were treated with ABT for another three days. The concentrations of SKF-525A that were tested were 100 µM and 200 µM; the concentrations of ABT that were tested were also 100 µM and 200 µM; the combinations tested are shown in Fig. 6. The results of these experiments (obtained on day 11 p.i.) are shown in Fig. 6, where Fig. 6A presents graphically that data in the table of Fig. 6B.

It was observed that treatment with 100 µM of SKF-525A and 200 µM of ABT gave maximal CYP1A2, CYP2C9, and CYP3A4 inhibition while not affecting cell viability or HBsAg secretion (Fig. 6B, boxed data).

### Example 5.4 - Evaluation of continuous treatment with SKF-525A and ABT

In this experiment, HBV-infected HepaRG cells were treated with SKF-525A and ABT for three days on day 5 post infection. On day 8 post infection, media containing SKF-525A and ABT was removed and cells were treated with SKF-525A and ABT for another three days. The concentration of SKF-525A was 2 µM and the concentrations of ABT that were tested were 100 µM and 200 µM; the combinations tested are shown in Fig. 7. Cell viability and HBsAg secretion were also monitored at day 11 p.i. The results of these experiments are shown in Fig. 7, where Fig. 7A presents graphically that data in the table of Fig. 7B.

In this experiment, it was observed that treatment with 2 µM of SKF-525A and 200 µM of ABT gave maximal CYP1A2, CYP2C9, and CYP3A4 inhibition while not affecting cell viability or HBsAg secretion (Fig. 7B, boxed data).

### Example 5.5 - Evaluation of ABT with furafylline (a CYP1A2 selective inhibitor)

In this experiment, the combination of ABT with the CYP1A2 selective inhibitor furafylline was evaluated to determine if the combination suppressed the activities of CYP1A2, CYP2C9, and CYP3A4 more effectively than either alone.

HBV-infected cells were exposed to 100 µM each of ABT and furafylline two times for three days each (days 5 and 8 p.i.; furafylline was previously titrated to determine that 100 µM was optimal under these conditions; furthermore, furafylline was observed to also inhibit CYP2C9). The activities of the indicated CYP enzymes were assayed (Fig. 11), and cell viability and HBsAg secretion monitored at 11 days p.i.

The results of this experiment are shown in Fig. 11. Fig. 11B shows the data in table form that are represented graphically in Fig. 11A. As shown, the combination of ABT and furafylline inhibited CYP1A2 by 94.6%,but CYP2C9 and CYP3A4 inhibition remained below 90%. Viability assay indicated that HepaRG cells viability was reducted by 11.6% after ABT and furafylline treatment.

### Example 5.6 - Evaluation of ABT with ketoconazole (a CYP3A4 selective inhibitor)

In this experiment, the combination of ABT with the CYP3A4 "selective" inhibitor ketoconazole was evaluated to determine if the combination suppressed the activities of CYP1A2, CYP2C9, and CYP3A4 more effectively than either alone.

HBV-infected cells were exposed to 100 µM of ABT and 5 µM ketoconazole two times for three days each (at days 5 and 8 p.i.; ketoconazole was previously titrated to determine that 5 µM was optimal under these conditions; furthermore, ketoconazole was observed to also inhibit CYP2C9). The activities of the indicated CYP enzymes were assayed, and cell viability and HBsAg secretion monitored.

The results of this experiment are shown in Fig. 12. Fig. 12B shows the data in table form that are represented graphically in Fig. 12A. As shown, the combination of ABT and ketoconazole improved CYP enzyme inhibition, although CYP2C9 and CYP1A2 remained below 90% inhibition.

### Example 6- Assay development: ABT and SKF-525A combination treatments in HBV-infected HepaRG cells

In these examples, three combination options were tested:
(1) sequential application of SKF-525A for one hour at day 5 p.i., then ABT applied twice for three days each (applied at days 5 and 8 p.i.; ABT would be co-dosed with test agents);
(2) continuous application of SKF-525A and ABT in combination, applied twice for three days each (applied at days 5 and 8 p.i.; SKF and ABT would be co-dosed with test agents); and
(3) pre-dosing of SKF-525A and ABT in combination for one hour, applied twice (applied at days 5 and 8 p.i.); the CYP inhibitors would be removed before adding test agents.

### Example 6.1 - Continuous treatment with SKF-525A and ABT

In this example, infected cells were treated with 2 µM of SKF-525A and either 100 µM or 200 µM ABT, applied twice for three days each (at days 5 and 8 p.i., see indicated tested combinations in Fig. 7). The inhibition of CYP1A2, CYP2C9, and CYP3A4 activities were assayed, and cell viability and HBsAg secretion monitored (at day 11 p.i.).

The results of this experiment are shown in Fig. 7. Fig. 7B shows the data in table form that are represented graphically in Fig. 7A. In this experiment, it was observed that treatment with 2 µM of SKF-525A and 200 µM of ABT gave maximal CYP1A2, CYP2C9, and CYP3A4 inhibition while not affecting cell viability or HBsAg secretion (Fig. 8B, boxed data). However, the inhibition of CYP2C9 was below 90% (85%, Fig. 8B).

The experiment was repeated to optimize CYP2C9 inhibition by increasing the concentration of SKF-525A to 5 µM and maintaining ABT at 200 µM; this condition was compared to 2 µM SKF-525A and 200 µM ABT.

The results of this experiment are shown in Fig. 8. Fig. 8A shows graphically the data presented in the table of Fig. 8B. In this experiment, it was observed that treatment with 5 µM of SKF-525A and 200 µM of ABT gave maximal CYP1A2, CYP2C9, and CYP3A4 inhibition while not affecting cell viability or HBsAg secretion (Fig. 8B, boxed data). Inhibition of CYP2C9 was almost at 90% (89.3%).

### Example 6.2 - Pre-dosing (before test agent application) with SKF-525A and ABT

In these experiments, the protocol of treating cells with SKF-525A and ABT (in combination) twice for one hour each at days 5 and 8 p.i. was evaluated.

In an initial experiment, SKF was used at 100 µM and ABT at 10 mM, but while CYP1A2, CYP2C9 and CYP3A4 activity were highly inhibited (96.8%, 95.5%, and 98.0%, respectively), and HBsAg secretion was minimally inhibited (1.3%), cell viability was also inhibited at 13.7% (all measured day 11 p.i.).

To optimize cell viability, the concentrations of SKF-525A was lowered to 0.1 mM and tested in combination with ABT at 5 mM and 10 mM, and the same assay repeated. The results of these experiments are shown in Fig. 9. Fig. 9B shows the data in table form that are represented graphically in Fig. 9A. In this experiment, it was observed that while under these condition, the CYP enzymes can be inhibited by greater than 90% (except for CYP1A2 treated with 100 µM SKF-525A and 5 mM ABT, the cells were observed under a microscope to have hepatocyte islands that appeared to be less healthy than untreated hepatocyte islands.

To further optimize cell viability, SKF-525A was tested at 25 µM and 50 µM, with ABT at either 5 mM or 10 mM. The results of these experiments are shown in Fig. 10. Figs. 10B and 10D show the data in table form that are represented graphically in Figs. 10A and 10C, respectively. From these data, it was observed that the combination to 50 µM SKF-525A and 10 mM ABT inhibited all three CYPs by at least 90% without affecting HBsAg secretion and cell viability (18D, boxed data). The method is detailed in Example 7.

### Example 7 - Restriction of metabolically active enzymes and antiviral test agent treatment of HBV-infected HepaRG cells (pre-dosing approach)

In order to suppress metabolism, HBV-infected HepaRG cells were incubated with Proadifen hydrochloride (SKF-525A) and 1-aminobenzotriazole (ABT). On day 5 p.i., SKF-525A and ABT were combined in DMSO, which was diluted 1:50 in HepaRG growth media and added to cells to achieve a final concentration of 10 mM ABT and 50 µM SKF-525A in 2% DMSO. CYP inhibitors were allowed to incubate with HBV-infected cells for 60 minutes at 37°C and 5% CO₂. After CYP inhibitors incubation, media containing SKF-525A and ABT was removed and test agents in DMSO were added at 1:50 to HepaRG cells and incubated for 3 days. On day 8 post infection, test agents were removed and cells were incubated with freshly prepared ABT and SKF-525A (final concentration of 10 mM ABT and 50 µM SKF-525A) for 60 minutes. After CYP inhibitors incubation, media containing SKF-525A and ABT was removed and test agents were added at 1:50 to HepaRG cells and incubated for an additional 3 days. Cells treated with 2% DMSO served as no test agent control and uninfected cells served as background signal. HBV analytical assays, CYP enzyme inhibition, and cell viability assays were performed on day 11 p.i., or after 6 days of test agent treatment.

### Example 8 - Evaluating test agents for HBV anti-viral activities

Three test agents with known stability were evaluated for HBV anti-viral activities using the assay as outlined in Example 7, with and without 50 µM SKF-525A and 10 mM ABT (in combination). Table 4 presents the tested test agents and the concentrations used:

**TABLE 4**

| | |
|---|---|
| Test agents and concentrations tested | |

| Test agent (TA) | Concentrations tested (in µM) |
|---|---|
| TA1 | 0.01,0.04, 0.1, 0.4, 1, 3.3, 10, 30 |
| TA2 | 0.002, 0.007, 0.03, 0.1, 0.5, 2, 7.5, 30 |
| TA3 | 0.01,0.04, 0.1, 0.4, 1, 3.3, 10, 30 |

The results of these experiments are shown in Fig. 13. As shown, TA1 (Fig. 13A), TA2 (Fig. 13B), and TA3 (Fig. 13C) exhibited "improved" antiviral activities in HBV-infected HepaRG cells when the cells had been treated with the combination of SKF-525 and ABT. HBV DNA is measured using branched DNA (bDNA) signal amplification. Table 5 presents data for the test agents showing EC₅₀ values for HepaRG cells and HepaRG cells treated with the CYP inhibitors.

**TABLE 5**

| | | | |
|---|---|---|---|
| Comparison of CYP inhibitor treated and untreated cells | | | |

| Test agent | Hepatocytes clearance (CL) values | HepaRG EC₅₀ | HepaRG - CYP inhibitor EC₅₀ |
|---|---|---|---|
| TA1 | 1.1 | 0.25 | 0.087 |
| TA2 | 81 | 0.56 | 0.0086 |
| TA3 | 99 | 1.6 | 0.15 |

### Example 9 - Evaluating effect of CYP inhibitors on restricting metabolic activities of PHH

Cryopreserved primary human hepatocytes (PHH donor 4038) were treated for one hour with or without Proadifen hydrochloride (SKF-525A) and 1-aminobenzotriazole (ABT) prior to infection. Pre-treated PHH and non-treated PHH were infected with HBV and allowed to incubate with CYP inhibitors for one hour on day 5 and day 8 post infection. HBV analytical assays, CYP enzyme inhibition, and cell viability assays were performed on day 11 p.i.. The results of these experiments are shown in Fig. 14. As shown, treatment of PHH donor 4038 with CYP inhibitors suppressed CYP3A4, 2C9 and 1A2 activities by >90% in PHH, without affecting cell viability and production of HBsAg (Fig. 14A).

Figure 15 details the results of experiments in cryopreserved primary human hepatocytes (PHH donor 4119E), treated with or without CYP inhibitors. In one condition, hepatocytes were first infected with HBV and then treated with CYP inhibitors (ABT at 10 mM and SKF-525A at 0.05 mM) for one hour on day 5 and day 8 post infection. In another condition, hepatocytes were first infected with HBV and then treated with CYP inhibitors (ABT at 10 mM and SKF-525A at 0.025 mM) for one hour on day 5 and day 8 post infection. HBV analytical assays, CYP enzyme inhibition, and cell viability assays were performed on day 11 p.i.. The results of these experiments are shown in Table 6 and Fig. 15.

| | SKF/ABT, 50uM/10mM | | | SKF/ABT, 25uM/10mM | | |
|---|---|---|---|---|---|---|
| | Mean | SD | N | Mean | SD | N |
| CYP1A2 | 90.0 | 9.34 | 4 | 80.2 | 5.73 | 2 |
| CYP3A4 | 96.9 | 1.43 | 4 | 87.5 | 6.93 | 2 |
| CYP2C9 | 70.4 | 9.16 | 2 | 60.4 | 4.45 | 2 |
| cell viability | 11.7 | 4.54 | 5 | -3.1 | 7.14 | 2 |
| HBsAg | 18.0 | 10.40 | 5 | 2.4 | 6.51 | 2 |
| HBV DNA | 12.6 | 11.02 | 4 | 0.7 | 6.15 | 2 |

Treatment of PHH donor 4119E with 50 uM SKF-525A and 10mM ABT resulted in >90% inhibition of CYP1A2 and CYP3A4 and 70.4% inhibition of CYP2C9 enzyme activities, while cell viability, HBsAg and HBV DNA production was reduced by 11.7%, 18.0% and 12.6%, respectively. Treatment of PHH donor 4119E with 25 uM SKF-525A and 10 mM ABT resulted in >80% inhibition for CYP1A2 and CYP3A4, and 60.4% inhibition for CYP2C9, while no significant reduction in cell viability or HBV replication was observed.

### Example 10 - Restriction of metabolically active enzymes and antiviral test agent treatment of HBV-infected Primary Human Hepatocytes (pre-dosing approach)

Two test agents were evaluated for HBV anti-viral activities in primary human hepatocytes (PHH donor 4038), with and without 50 µM SKF-525A and 10 mM ABT (in combination). Table 7 presents the tested test agents (TA) and the concentrations used:

| | |
|---|---|
| TABLE 7 | |
| Test agents and concentrations tested | |

| Test agent (TA) | Concentrations tested (in µM) |
|---|---|
| TA4 | 0.005, 0.014, 0.04, 0.1, 0.4, 1,3.3, 10, 30 |
| TA5 | 0.0002, 0.0007, 0.003, 0.005, 0.012, 0.014, 0.04, 0.05, 0.1, 0.19, 0.4, 0.75, 1, 3, 3.3, 10 |

PHH (donor 4038) were incubated with or without Proadifen hydrochloride (SKF-525A) and 1-aminobenzotriazole (ABT) for one hour, after which the CYP inhibitor containing media was removed. Media containing HBV inoculum and 1:50 diluted test agents (serial diluted in DMSO) were then added to PHH and allowed to incubate overnight. On day 1 post infection, media containing HBV and test agents was removed, cells were washed 3 times, and HBV-infected PHH were incubated again with CYP inhibitors for 60 minutes at 37°C and 5% CO₂. After CYP inhibitors incubation, media containing SKF-525A and ABT was removed and test agents diluted in DMSO were added at 1:50 dilution to PHH. On day 5 and day 8 post infection, media containing HBV and test agents was removed, and cells were incubated again with CYP inhibitors for 60 minutes at 37°C and 5% CO₂. After CYP inhibitors incubation, media containing SKF-525A and ABT was removed and test agents diluted in DMSO were added at 1:50 dilution to PHH. Cells treated with 2% DMSO served as no test agent control and uninfected cells served as background signal. HBV DNA was measured using branched DNA (bDNA) signal amplification on day 11 p.i. The results of these experiments are shown in Figure 16. As shown, TA4 (Fig. 16A) and TA5 (Fig. 16B) exhibited "improved" antiviral activities in HBV-infected PHH when the cells had been treated with SKF-525 and ABT. Table 8 presents data for the test agents showing EC₅₀ values for PHH with or without the CYP inhibitors.

**TABLE 8**

| | | |
|---|---|---|
| Comparison of CYP inhibitor treated and untreated cells | | |

| Test agent | PHH EC₅₀ | PHH - CYP inhibitor EC₅₀ |
|---|---|---|
| TA4 | 3.3 | 0.19 |
| TA5 | 0.048 | 0.004 |

## Claims

1. A method of identifying a hepatitis B virus (HBV) antiviral agent in an assay, *in vitro,* wherein the assay comprises using an HBV-infected cell, wherein the metabolic capacity of the HBV-infected cell is modulated, and applying a test agent to the HBV-infected cell such that the test agent is not metabolized or is minimally metabolized during the assay.

2. A method according to claim 1, wherein the assay comprises applying a test agent to an HBV-infected cell, wherein the HBV-infected cell is treated with one or more cytochrome P450 inhibitors that modulate or inhibit the metabolic capacity of the HBV-infected cell such that the test agent is not metabolized or is minimally metabolized during the assay, and wherein cell viability and an assay endpoint are measured, and wherein the assay endpoint is selected from the group consisting of antiviral innate immune genes expression and/or protein production, antiviral gene expression and/or protein production, HBV cccDNA, degraded HBV cccDNA, transcription of HBV cccDNA, nuclear import of HBV polypeptides or HBV DNA, nuclear export of HBV polypeptides or HBV DNA, a function of HBV polypeptide, a function of HBV core polypeptide, HBV capsid polypeptide assembly, HBV replication, HBV genome encapsidation, HBV genome replication, HBV RNA encapsidation, HBV RNA secretion, HBV RNA splicing, HBV virion morphogenesis, HBV virion secretion, the presence of HBsAg, and the presence of HBeAg antigen.

3. A method according to claim 1, wherein the assay comprises applying a test agent to an HBV-infected cell, wherein the metabolic capacity of the HBV-infected cell is modulated such that the test agent is not metabolized or is minimally metabolized during the assay, comprising the steps of, in any order:
(a) treating a cell with one or more enzyme modulators that modulate the metabolic capacity of the cell;
(b) infecting the cell with HBV; and
(c) applying the test agent to the HBV-infected cell.

4. The method of claim 3, wherein an assay endpoint is maintainedwherein the assay endpoint is selected from the group consisting of antiviral innate immune genes expression and/or protein production, antiviral gene expression and/or protein production, HBV cccDNA, degraded HBV cccDNA, transcription of HBV cccDNA, nuclear import of HBV polypeptides or HBV DNA, nuclear export of HBV polypeptides or HBV DNA, a function of HBV polypeptide, a function of HBV core polypeptide, HBV capsid polypeptide assembly, HBV replication, HBV genome encapsidation, HBV genome replication, HBV RNA encapsidation, HBV RNA secretion, HBV RNA splicing, HBV virion morphogenesis, HBV virion secretion, HBsAg, and HBeAg antigen.

5. The method of claim 3, wherein the test agent is identified as a hepatitis B virus (HBV) antiviral agent when the appropriate HBV assay endpoints are compared to a control cell in the assay lacking the test agent.

6. The method of claim 3, wherein the one or more enzyme modulators is a cytochrome P450 inhibitor.

7. The method of claim 6, wherein the one or more cytochrome P450 inhibitors comprise SKF-525A and 1-aminobenzotriazole.

8. The method of claim 3, wherein the cell is a human cell.

9. The method of claim 8, wherein the cell is from a cell line or a primary culture cell.

10. The method of claim 9, wherein the cell is from a cell line and is a HepaRG cell or a primary human hepatocyte.

11. A method of identifying an antiparasitic agent in an assay, *in vitro,* wherein the assay comprises applying a test agent to a *Plasmodium-*infected cell, wherein the metabolic capacity of the *Plasmodium-*infected cell is modulated such that the test agent is not metabolized or is minimally metabolized during the assay, comprising the steps of, in any order:
(a) treating a cell with one or more enzyme modulators that modulate the metabolic capacity of the cell;
(b) infecting the cell with *Plasmodium;* and
(c) applying the test agent to the *Plasmodium-*infected cell.

12. A method of identifying an antidiabetic agent in an assay, *in vitro,* wherein the assay comprises applying a test agent to a cell, wherein the metabolic capacity of the cell is modulated such that the test agent is not metabolized or is minimally metabolized during the assay, comprising the steps of, in any order:
(a) treating a cell with one or more enzyme modulators that modulate the metabolic capacity of the cell; and
(b) applying the test agent to the cell.

13. A combination comprising at least one metabolizing cell; at least one enzyme modulator; and at least one test agent metabolized by said metabolizing cell, wherein the metabolizing cell is an hepatocyte infected with HBV.

14. The combination of claim 13, wherein the test agent is an antiviral agent

15. A combination comprising at least one metabolizing cell; at least one enzyme modulator; and at least one test agent metabolized by said metabolizing cell, wherein the metabolizing cell is an hepatocyte infected with *Plasmodium.*

16. The combination of claim 15, wherein the test agent is an anti-parasitic agent

17. A combination comprising at least one metabolizing cell; at least one enzyme modulator; and at least one test agent metabolized by said metabolizing cell, wherein the test agent is an antidiabetic agent.

18. The method of claim 1 comprising:
(a) treating a metabolizing cell with one or more enzyme modulators;
(b) infecting the cell with HBV;
(c) applying a test agent to the HBV-infected cell; and
(d) determining if the test agent achieves an HBV endpoint, wherein achievement of the HBV endpoint identifies the test agent as an HBV antiviral agent, wherein the HBV endpoint is selected from the group consisting of antiviral innate immune genes expression and/or protein production, antiviral gene expression and/or protein production, HBV cccDNA, degraded HBV cccDNA, transcription of HBV cccDNA, nuclear import of HBV polypeptides or HBV DNA, nuclear export of HBV polypeptides or HBV DNA, a function of HBV polypeptide, a function of HBV core polypeptide, HBV capsid polypeptide assembly, HBV replication, HBV genome encapsidation, HBV genome replication, HBV RNA encapsidation, HBV RNA secretion, HBV RNA splicing, HBV virion morphogenesis, HBV virion secretion, the presence of HBsAg, and the presence of HBeAg antigen.

## Patentansprüche

1. Verfahren zur Identifizierung eines HBV(Hepatitis B Virus)-Antivirusmittels in einem Test, *in vitro,* wobei der Test Verwenden einer HBV-infizierten Zelle, wobei die metabolische Kapazität der HBV-infizierten Zelle moduliert wird, und Anwenden eines Testagens auf die HBV-infizierte Zelle, so dass das Testagens während des Tests nicht oder minimal metabolisiert wird, umfasst.

2. Verfahren gemäß Anspruch 1, wobei der Test Anwenden eines Testagens auf eine HBV-infizierte Zelle umfasst, wobei die HBV-infizierte Zelle mit einem oder mehreren Cytochrom-P450-Inhibitoren behandelt wird, die die metabolische Kapazität der HBV-infizierten Zelle modulieren oder hemmen, so dass das das Testagens während des Tests nicht oder minimal metabolisiert wird, und wobei Zellviabilität und ein Testendpunkt gemessen werden und wobei der Testendpunkt ausgewählt ist aus der Gruppe bestehend aus antiviraler angeborener Immungene-Expression und/oder -Proteinproduktion, antiviraler Genexpression und/oder Proteinproduktion, HBV-cccDNA, abgebauter HBV-cccDNA, Transkription von HBV-cccDNA, Kernimport von HBV-Polypeptiden oder HBV-DNA, Kernexport von HBV-Polypeptiden oder HBV-DNA, einer Funktion von HBV-Polypeptid, einer Funktion von HBV-Core-Polypeptid, HBV-Kapsidpolypeptidzusammenbau, HBV-Replikation, HBV-Genom-Verkapselung, HBV-Genom-Replikation, HBV-RNA-Verkapselung, HBV-RNA-Sekretion, HBV-RNA-Spleißen, HBV-Virion-Morphogenese, HBV-Virion-Sekretion, dem Vorliegen von HBsAg und dem Vorliegen von HBeAg-Antigen.

3. Verfahren gemäß Anspruch 1, wobei der Test Anwenden eines Testagens auf eine HBV-infizierte Zelle umfasst, wobei die metabolische Kapazität der HBV-infizierten Zelle moduliert wird, so dass das Testagens während des Tests nicht oder minimal metabolisiert wird, umfassend in beliebiger Reihenfolge die Schritte:
(a) Behandeln einer Zelle mit einem oder mehreren Enzymmodulatoren, die die metabolische Kapazität der Zelle modulieren;
(b) Infizieren der Zelle mit HBV; und
(c) Anwenden des Testagens auf die HBV-infizierte Zelle.

4. Verfahren nach Anspruch 3, wobei ein Testendpunkt erhalten wird, wobei der Testendpunkt ausgewählt ist aus der Gruppe bestehend aus antiviraler angeborener Immungene-Expression und/oder -Proteinproduktion, antiviraler Genexpression und/oder Proteinproduktion, HBV-cccDNA, abgebauter HBV-cccDNA, Transkription von HBV-cccDNA, Kernimport von HBV-Polypeptiden oder HBV-DNA, Kernexport von HBV-Polypeptiden oder HBV-DNA, einer Funktion von HBV-Polypeptid, einer Funktion von HBV-Core-Polypeptid, HBV-Kapsidpolypeptidzusammenbau, HBV-Replikation, HBV-Genom-Verkapselung, HBV-Genom-Replikation, HBV-RNA-Verkapselung, HBV-RNA-Sekretion, HBV-RNA-Spleißen, HBV-Virion-Morphogenese, HBV-Virion-Sekretion, HBsAg und HBeAg-Antigen.

5. Verfahren nach Anspruch 3, wobei das Testagens als ein HBV(Hepatitis B Virus)-Antivirusmittel identifiziert wird, wenn die entsprechenden HBV-Testendpunkte mit einer Kontrollzelle im Test ohne das Testagens verglichen werden.

6. Verfahren nach Anspruch 3, wobei es sich bei den ein oder mehreren Enzymmodulatoren um einen Cytochrom-P450-Inhibitor handelt.

7. Verfahren nach Anspruch 6, wobei die ein oder mehreren Cytochrom-P450-Inhibitoren SKF-525A und 1-Aminobenzotriazol umfassen.

8. Verfahren nach Anspruch 3, wobei es sich bei der Zelle um eine menschliche Zelle handelt.

9. Verfahren nach Anspruch 8, wobei die Zelle von einer Zelllinie stammt oder eine Primärkulturzelle ist.

10. Verfahren nach Anspruch 9, wobei die Zelle von einer Zelllinie stammt und es sich dabei um eine HepaRG-Zelle oder einen primären menschlichen Hepatozyten handelt.

11. Verfahren zur Identifizierung eines Mittels gegen Parasiten in einem Test, *in vitro,* wobei der Test Anwenden eines Testagens auf eine mit *Plasmodium* infizierte Zelle umfasst, wobei die metabolische Kapazität der mit *Plasmodium* infizierten Zelle moduliert wird, so dass das Testagens während des Tests nicht oder minimal metabolisiert wird, umfassend in beliebiger Reihenfolge die Schritte:
(a) Behandeln einer Zelle mit einem oder mehreren Enzymmodulatoren, die die metabolische Kapazität der Zelle modulieren;
(b) Infizieren der Zelle mit *Plasmodium;* und
(c) Anwenden des Testagens auf die mit *Plasmodium* infizierte Zelle.

12. Verfahren zur Identifizierung eines Mittels gegen Diabetes in einem Test, *in vitro,* wobei der Test Anwenden eines Testagens auf eine Zelle umfasst, wobei die metabolische Kapazität der Zelle moduliert wird, so dass das Testagens während des Tests nicht oder minimal metabolisiert wird, umfassend in beliebiger Reihenfolge die Schritte:
(a) Behandeln einer Zelle mit einem oder mehreren Enzymmodulatoren, die die metabolische Kapazität der Zelle modulieren; und
(b) Anwenden des Testagens auf die Zelle.

13. Kombination, umfassend wenigstens eine metabolisierende Zelle; wenigstens einen Enzymmodulator; und wenigstens ein von der metabolisierenden Zelle metabolisiertes Testagens, wobei es sich bei der metabolisierenden Zelle um einen mit HBV infizierten Hepatozyten handelt.

14. Kombination nach Anspruch 13, wobei es sich bei dem Testagens um ein Antivirusmittel handelt.

15. Kombination, umfassend wenigstens eine metabolisierende Zelle; wenigstens einen Enzymmodulator; und wenigstens ein von der metabolisierenden Zelle metabolisiertes Testagens, wobei es sich bei der metabolisierenden Zelle um einen mit *Plasmodium* infizierten Hepatozyten handelt.

16. Kombination nach Anspruch 15, wobei es sich bei dem Testagens um ein Mittel gegen Parasiten handelt.

17. Kombination, umfassend wenigstens eine metabolisierende Zelle; wenigstens einen Enzymmodulator; und wenigstens ein von der metabolisierenden Zelle metabolisiertes Testagens, wobei es sich bei dem Testagens um ein Mittel gegen Diabetes handelt.

18. Verfahren nach Anspruch 1, umfassend:
(a) Behandeln einer metabolisierenden Zelle mit einem oder mehreren Enzymmodulatoren;
(b) Infizieren der Zelle mit HBV;
(c) Anwenden eines Testagens auf die HBVinfizierte Zelle; und
(d) Bestimmen, ob durch das Testagens ein HBV-Endpunkt erreicht wird, wobei durch Erreichen des HBV-Endpunkts das Testagens als ein HBV-Antivirusmittel identifiziert wird, wobei der HBV-Endpunkt ausgewählt ist aus der Gruppe bestehend aus antiviraler angeborener Immungene-Expression und/oder -Proteinproduktion, antiviraler Genexpression und/oder Proteinproduktion, HBV-cccDNA, abgebauter HBV-cccDNA, Transkription von HBV-cccDNA, Kernimport von HBV-Polypeptiden oder HBV-DNA, Kernexport von HBV-Polypeptiden oder HBV-DNA, einer Funktion von HBV-Polypeptid, einer Funktion von HBV-Core-Polypeptid, HBV-Kapsidpolypeptidzusammenbau, HBV-Replikation, HBV-Genom-Verkapselung, HBV-Genom-Replikation, HBV-RNA-Verkapselung, HBV-RNA-Sekretion, HBV-RNA-Spleißen, HBV-Virion-Morphogenese, HBV-Virion-Sekretion, dem Vorliegen von HBsAg und dem Vorliegen von HBeAg-Antigen.

## Revendications

1. Procédé d'identification d'un agent antiviral contre le virus de l'hépatite B (VHB) dans un dosage, *in vitro,* dans lequel le dosage comprend les étapes consistant à utiliser une cellule infectée par VHB, dans lequel la capacité métabolique de la cellule infectée par VHB est modulée, et appliquer un agent de test à la cellule infectée par VHB de sorte que l'agent de test ne soit pas métabolisé ou soit métabolisé de manière minimale pendant le dosage.

2. Procédé selon la revendication 1, dans lequel le dosage comprend l'étape consistant à appliquer un agent de test à une cellule infectée par VHB, dans lequel la cellule infectée par VHB est traitée avec un ou plusieurs inhibiteur(s) du cytochrome P450 qui module/modulent ou inhibe/inhibent la capacité métabolique de la cellule infectée par VHB de sorte que l'agent de test ne soit pas métabolisé ou soit métabolisé de manière minimale pendant le dosage, et dans lequel on mesure la viabilité cellulaire et un point final du dosage, et dans lequel le point final du dosage est choisi dans le groupe constitué par l'expression de gènes et/ou la production de protéines de l'immunité innée antivirale, l'expression de gènes et/ou la production de protéines antiviraux, l'ADNccc de VHB, l'ADNccc de VHB dégradé, la transcription de l'ADNccc de VHB, l'importation nucléaire de polypeptides de VHB ou d'ADN de VHB, l'exportation nucléaire de polypeptides de VHB ou d'ADN de VHB, une fonction de polypeptide de VHB, une fonction de polypeptide du noyau de VHB, un assemblage de polypeptides de la capside de VHB, la réplication de VHB, l'encapsidation du génome de VHB, la réplication du génome de VHB, l'encapsidation d'ARN de VHB, la sécrétion d'ARN de VHB, l'épissage d'ARN de VHB, la morphogenèse du virion de VHB, la sécrétion du virion de VHB, la présence d'un HBsAg et la présence d'un antigène HBeAg.

3. Procédé selon la revendication 1, dans lequel le dosage comprend l'étape consistant à appliquer un agent de test à une cellule infectée par VHB, dans lequel la capacité métabolique de la cellule infectée par VHB est modulée de sorte que l'agent de test ne soit pas métabolisé ou soit métabolisé de manière minimale pendant le dosage, comprenant dans n'importe quel ordre les étapes consistant à :
(a) traiter une cellule avec un ou plusieurs modulateur(s) enzymatique (s) qui module/modulent la capacité métabolique de la cellule ;
(b) infecter la cellule avec un VHB ; et
(c) appliquer l'agent de test à la cellule infectée par VHB.

4. Procédé de la revendication 3, dans lequel un point final du dosage est maintenu, dans lequel le point final du dosage est choisi dans le groupe constitué par l'expression de gènes et/ou la production de protéines de l'immunité innée antivirale, l'expression de gènes et/ou la production de protéines antiviraux, l'ADNccc de VHB, l'ADNccc de VHB dégradé, la transcription de l'ADNccc de VHB, l'importation nucléaire de polypeptides de VHB ou d'ADN de VHB, l'exportation nucléaire de polypeptides de VHB ou d'ADN de VHB, une fonction de polypeptide de VHB, une fonction de polypeptide du noyau de VHB, un assemblage de polypeptides de la capside de VHB, la réplication de VHB, l'encapsidation du génome de VHB, la réplication du génome de VHB, l'encapsidation d'ARN de VHB, la sécrétion d'ARN de VHB, l'épissage d'ARN de VHB, la morphogenèse du virion de VHB, la sécrétion du virion de VHB, un HBsAg et un antigène HBeAg.

5. Procédé de la revendication 3, dans lequel l'agent de test est identifié comme étant un agent antiviral contre le virus de l'hépatite B (VHB) quand les points finaux appropriés du dosage de VHB sont comparés avec une cellule témoin dans le dosage dépourvu de l'agent de test.

6. Procédé de la revendication 3, dans lequel le ou les plusieurs modulateur (s) enzymatique(s) est/sont un inhibiteur du cytochrome P450.

7. Procédé de la revendication 6, dans lequel le ou les plusieurs inhibiteur(s) du cytochrome P450 comprend/comprennent des SKF-525A et 1-aminobenzotriazole.

8. Procédé de la revendication 3, dans lequel la cellule est une cellule humaine.

9. Procédé de la revendication 8, dans lequel la cellule provient d'une lignée de cellules ou d'une cellule de culture primaire.

10. Procédé de la revendication 9, dans lequel la cellule provient d'une lignée de cellules et est une cellule HepaRG ou un hépatocyte primaire humain.

11. Procédé d'identification d'un agent antiparasitaire dans un dosage, *in vitro,* dans lequel le dosage comprend l'étape consistant à appliquer un agent de test à une cellule infectée par *Plasmodium,* dans lequel la capacité métabolique de la cellule infectée par *Plasmodium* est modulée de sorte que l'agent de test ne soit pas métabolisé ou soit métabolisé de manière minimale pendant le dosage, comprenant dans n'importe quel ordre les étapes consistant à :
(a) traiter une cellule avec un ou plusieurs modulateur(s) enzymatique (s) qui module/modulent la capacité métabolique de la cellule ;
(b) infecter la cellule avec *Plasmodium* ; et
(c) appliquer l'agent de test à la cellule infectée par *Plasmodium.*

12. Procédé d'identification d'un agent antidiabétique dans un dosage, *in vitro,* dans lequel le dosage comprend l'étape consistant à appliquer un agent de test à une cellule, dans lequel la capacité métabolique de la cellule est modulée de sorte que l'agent de test ne soit pas métabolisé ou soit métabolisé de manière minimale pendant le dosage, comprenant dans n'importe quel ordre les étapes consistant à :
(a) traiter une cellule avec un ou plusieurs modulateur(s) enzymatique (s) qui module/modulent la capacité métabolique de la cellule ; et
(b) appliquer l'agent de test à la cellule.

13. Combinaison comprenant au moins une cellule de métabolisation ; au moins un modulateur enzymatique ; et au moins un agent de test métabolisé par ladite cellule de métabolisation, dans laquelle la cellule de métabolisation est un hépatocyte infecté par un VHB.

14. Combinaison de la revendication 13, dans laquelle l'agent de test est un agent antiviral.

15. Combinaison comprenant au moins une cellule de métabolisation ; au moins un modulateur enzymatique ; et au moins un agent de test métabolisé par ladite cellule de métabolisation, dans laquelle la cellule de métabolisation est un hépatocyte infecté par *Plasmodium.*

16. Combinaison de la revendication 15, dans laquelle l'agent de test est un agent antiparasitaire.

17. Combinaison comprenant au moins une cellule de métabolisation ; au moins un modulateur enzymatique ; et au moins un agent de test métabolisé par ladite cellule de métabolisation, dans laquelle l'agent de test est un agent antidiabétique.

18. Procédé de la revendication 1, comprenant les étapes consistant à :
(a) traiter une cellule de métabolisation avec un ou plusieurs modulateur(s) enzymatique(s) ;
(b) infecter la cellule avec un VHB ;
(c) appliquer un agent de test à la cellule infectée par VHB ; et
(d) déterminer si l'agent de test atteint un point final de VHB, dans lequel le fait d'atteindre le point final de VHB identifie l'agent de test comme étant un agent antiviral contre le VHB, dans lequel le point final de VHB est choisi dans le groupe constitué par l'expression de gènes et/ou la production de protéines de l'immunité innée antivirale, l'expression de gènes et/ou la production de protéines antiviraux, l'ADNccc de VHB, l'ADNccc de VHB dégradé, la transcription de l'ADNccc de VHB, l'importation nucléaire de polypeptides de VHB ou d'ADN de VHB, l'exportation nucléaire de polypeptides de VHB ou d'ADN de VHB, une fonction de polypeptide de VHB, une fonction de polypeptide du noyau de VHB, un assemblage de polypeptides de la capside de VHB, la réplication de VHB, l'encapsidation du génome de VHB, la réplication du génome de VHB, l'encapsidation d'ARN de VHB, la sécrétion d'ARN de VHB, l'épissage d'ARN de VHB, la morphogenèse du virion de VHB, la sécrétion du virion de VHB, la présence d'un HBsAg et la présence d'un antigène HBeAg.
